# EUROPEAN PATENT SPECIFICATION

(11) **EP 4 507 757 B1**
(45) Date of publication and mention of the grant of the patent: **12.08.2026**
(21) Application number: 23706350.8
(22) Date of filing: 21.02.2023
(51) Int. Cl.: A61M 5/142, A61M 5/32

(54) **PATCH-LIKE MEDICAL DEVICE WITH MOVABLE NEEDLE SLEEVE**
PFLASTERARTIGE MEDZINISCHE VORRICHTUNG MIT BEWEGLICHER NADELSCHUTZHÜLSE
DISPOSITIF MÉDICAL DE TYPE PATCH AVEC GAINE MOBILE DE PROTECTION D'AIGUILLE

(30) Priority: 14.04.2022 EP 22168319; 17.06.2022 WO PCT/EP2022/066589
(43) Date of publication of application: 19.02.2025
(60) Divisional application: 25226802.4 / 4 696 346; 25226805.7 / 4 710 971; 25226812.3 / 4 696 347
(73) Proprietor: PharmaSens AG, 2503 Biel/Bienne (CH)
(72) Inventor: LODICO, Tamara, 5014 Gretzenbach (CH); TEUTSCH, David, 3036 Detligen (CH); SCHRANZ, Samuel, 3252 Worben (CH); BOTH, Marcel, 3038 Kirchlindach (CH); MCDONALD, Jérôme, 6340 Baar (CH); PFAFF, Jonas, 8610 Uster (CH); MEIER, Timon, 8815 Horgenberg (CH); RITTER, Moritz, 8046 Zürich (CH)
(74) Representative: Mayer, Aline Sophie
(86) International application number: PCT/EP2023/054336
(87) International publication number: WO 2023/198343

(56) References cited:
- EP-A1- 2 650 032
- EP-A1- 3 260 146
- WO-A1-2009/013735
- WO-A1-2009/098291
- WO-A2-02/068015
- WO-A2-2013/134486
- US-A- 5 814 020
- US-A- 5 931 814
- US-A1- 2002 022 798
- US-A1- 2011 213 329
- US-A1- 2014 035 604
- US-A1- 2016 263 312
- US-A1- 2018 185 572
- US-A1- 2019 248 518

## Description

### TECHNICAL FIELD

The present invention relates to a patch-like medical device configured to be applied to a patient's skin.

### PRIOR ART

Certain medical conditions, such as for instance diabetes, require regular administration of a liquid substance, such as insulin, into a patient's body through the patient's skin. To facilitate administration, patch-like medical devices are known, which are configured to be applied to the patient's skin and preferably to be worn by the patient over an extended period of time. Such patch-like devices generally comprise a hollow needle (cannula) configured to pierce the patient's skin and through which needle the liquid substance is being delivered into the patient's body. In particular if the liquid substance is to be administered slowly and/or regularly over an extended period of time, the needle is advantageously connected to a delivery system comprising an automatic pump mechanism. In order to be able to pierce the patient's skin when the patch-like medical device is applied to the body, the needle preferably has a sharp needle tip which protrudes from said contact surface of the patch-like medical device. However, before the patch-like medical device is applied to the patient, this needle tip needs to be protected from damage, and any person handling the patch-like medical device prior to use needs to be protected from accidently getting injured by the protruding needle tip.

US5931814A discloses an injection device having a casing containing an active substance reservoir, a cannula communicating with the reservoir, a device for inserting the cannula, and pump means for discharging the reservoir contents through the cannula. The cannula is fixed relative to the casing and projects beyond the underside of the casing to the depth required for injection. The cannula is surrounded by a protective element which is moved by a spring drive from a first end position, in which the protective device projects beyond the underside of the casing and beyond the cannula, to a second end position in which the protective device does not project beyond the underside of the casing. In particular, a cylindrical protective sleeve is disclosed, which is dimensioned and disposed so that in the inoperative position it projects sufficiently beyond the cannula to prevent accidental contact with the cannula tip. To insert the cannula, the protective sleeve is retracted by spring force into the casing.

Since the needle has to protrude beyond the underside of the casing by a length which is sufficient to achieve a required injection depth, the protective sleeve has to extend over said length as well to fulfill its protective purpose. As a consequence, said length defines a minimum thickness the casing needs to have in order to provide sufficient space for the protective sleeve to be fully received within the casing when the protective sleeve is retracted.

For the patient however, an increase of the casing thickness negatively impacts the comfort.

### SUMMARY OF THE INVENTION

In a first aspect, it is an object of the present invention to provide a patch-like medical device with a needle protection mechanism that allows the patient's skin to be pierced easily, while being compact and thus allowing for the patch-like medical device to be slim, which in turn enhances the patient's comfort.

This object is achieved by a patch-like medical device according to claim 1. Further embodiments of the invention are laid down in the dependent claims.

The present invention provides a patch-like medical device comprising:
a base having a contact surface configured to be directed towards to a patient's skin;
a needle fixedly positioned relative to the base, the needle having a needle tip which protrudes from the contact surface and which is configured for piercing the patient's skin, and
a needle protection sleeve movable along a central axis from a first position to a second position, the needle protection sleeve extending beyond the needle tip to prevent the needle tip from piercing the patient's skin in the first position, and exposing the needle tip to enable piercing the patient's skin in the second position.

The patch-like medical device of the present invention further comprises a rotating sleeve movable along the central axis, the rotating sleeve being in engagement with the needle protection sleeve such that a helical movement of the rotating sleeve with respect to the base causes the needle protection sleeve to move along the central axis from the first position to the second position.

Furthermore, patch-like medical device of the present invention comprises a rotation lock sleeve movable along the central axis while being rotationally fixed with respect to the base, the rotation lock sleeve being in engagement with the needle protection sleeve such that the needle protection sleeve is prevented from rotating with respect to the base when the needle protection sleeve moves from the first position to the second position.

The term "needle" as used herein is equivalent to the term "infusion needle" or "cannula" and refers to any elongated sharp element configured to pierce the patient's skin and preferably having a lumen for delivering a liquid substance, in particular insulin, through the patient's skin.

The central axis preferably coincides with an injection axis (longitudinal axis) defined by the needle and is preferably perpendicular to the contact surface. In the present context, the term "distal" refers to a direction parallel to the central axis leading away from the contact surface, i.e. towards the patient's skin when the patch-like medical device is applied as intended. The term "proximal" refers to a direction which is 180° opposite to the distal direction, i.e. leading away from the patient's skin when the patch-like medical device is applied as intended. According to this definition, the needle protection sleeve moves in proximal direction when moving from the first position to the second position.

Preferably, the needle protection sleeve, the rotating sleeve and the rotation lock sleeve are concentrically arranged around the central axis.

In the first position, the rotating sleeve and/or the rotation lock sleeve preferably at least partially protrudes from the contact surface in the distal direction, and the needle protection sleeve preferably telescopically protrudes beyond the rotating sleeve and the rotation lock sleeve.

The contact surface may comprise an adhesive layer or suction elements for direct adhesion to the patient's skin. Alternatively, the contact surface may be configured to be attached to an adhesive sheet (plaster), wherein the sheet is in turn configured to be in direct contact with the patient's skin. In such a case, the needle protection sleeve, the rotating sleeve and the rotation lock sleeve are preferably not covered by said sheet, i.e. the sheet is preferably ring-shaped with a central hole arranged such that, in the first position, the sleeves may protrude from the contact surface through said hole.

Having the rotating sleeve perform a helical movement while being in engagement with the needle protection sleeve and having a rotation lock sleeve being in engagement with the needle protection sleeve enables a telescopic interaction between the rotating sleeve, the needle protection sleeve and the rotation lock sleeve and thus provides a compact needle protection solution.

The needle protection sleeve and the rotating sleeve are in a thread-like engagement with each other.

In the present context, a "thread-like engagement" refers to a mechanical engagement between a first part and a second part that causes a screwing motion, i.e. a combined axial and rotational movement, of the first part relative to the second part. In particular, a thread-like engagement may involve a thread, for instance in form of a helical groove, on the first part and a thread or thread portions, for instance in form of helical ribs, on the second part, the thread portions of the second part being configured to engage with said thread of the first part. The threads and the thread portions mentioned in this disclosure may in particular be square threads, i.e. have a square profile.

The rotating sleeve preferably at least partially protrudes from the contact surface when the needle protection sleeve is in the first position, wherein the rotating sleeve axially retracts towards the contact surface, i.e. in proximal direction, along the central axis during its helical movement, and wherein the needle protection sleeve is configured to axially move relative to the rotating sleeve along the central axis during the helical movement of the rotating sleeve. The rotation lock sleeve may be entrained to axially retract towards the contact surface by the rotating sleeve or by the needle protection sleeve during the helical movement of the rotating sleeve.

In the second position, the needle protection sleeve, the rotating sleeve and the rotation lock sleeve may each be at least partially, preferably fully, retracted inside the patch-like medical device on a side that faces away from the contact surface. In the present context, "fully retracted" means that neither the needle protection sleeve nor the rotating sleeve nor the rotation lock sleeve are protruding from the contact surface, which represents a preferred state for the second position in terms of comfort for the patient.

Due to the telescopic interaction between the rotating sleeve, the needle protection sleeve and the rotation lock sleeve, each of these sleeves may have a length in axial direction with respect to the central axis that is shorter than a needle protrusion length by which the needle protrudes from the contact surface towards the patient's skin, the sleeves thus taking up less space within the patch-like medical device when retracted, thereby allowing the patch-like medical device to be slimmer in axial direction and thus more comfortable for the patient.

The base may form a housing lower part and the patch-like medical device may comprise a housing upper part connected to the base. In some embodiments, the base may simply have a through-hole, and the sleeves may be received in a cavity formed between the base and the housing upper part. Preferably however, the base may define a recess on the side that faces away from the contact surface for receiving the sleeves. The recess may be delimited in axial direction by a top wall. In radial direction, the recess may be delimited by a side wall, the side wall being preferably cylindrical. The top wall may have bore in which the needle is held. An axial protrusion may be present around the needle, the axial protrusion extending distally from the top wall into the recess. The axial protrusion may act to provide the needle with added stability against lateral tilting and bending movements. In other embodiments, the needle may be held by any other type of needle holding structure that is fixedly connected to the base. The medical patch-like device preferably comprises a reservoir configured to contain a liquid substance, preferably insulin. The needle may be fluidically connected to the reservoir. The medical patch-like device may comprise a pump mechanism causing the liquid substance to be pumped from the reservoir through the needle. The pump mechanism and the reservoir are preferably arranged on the side of the base facing away from the contact surface and are ideally protected by the housing upper part. The housing upper part may be configured to be easily removable from the base to enable a refill or exchange of the reservoir. Alternatively, the base and the housing upper part may be permanently sealed.

Preferably, the patch-like medical device comprises a first device part which is disposable, i.e. intended for one-time use, and a second device part which is reusable, i.e. intended for multiple use. Preferably the first device part comprises the base, the reservoir for containing the liquid substance, as well as the needle and the needle protection mechanism, and the second device part comprises the housing upper part.

In the patch-like medical device disclosed here, the needle is fixedly positioned with respect to the contact surface of the base, which provides the advantage that the patient may comfortably move while wearing the patch-like medical device without causing a flow of the liquid substance to be inadvertently interrupted by an accidental movement of the needle.

In particular, the needle protection sleeve may have, at a distal end, a protection sleeve front wall defining a protection sleeve contact surface, which is in contact with the patient's skin when the patch-like medical device is applied to the patient. The protection sleeve contact surface may comprise an adhesive layer or suction elements to help securing the patch-like medical device to the patient's skin.

The protection sleeve front wall may have a needle hole, through which needle hole the needle tip protrudes when the needle protection sleeve is in the second position. Preventing the needle protection sleeve from rotating with respect to the base thus ensures that the patient's skin is not getting twisted while the needle protection sleeve is moving from the first position to the second position. When first applying the medical patch-like device to the patient's skin, the patient's skin may be slightly pushed in by the needle protection sleeve. While the needle protection sleeve is moving from the first position to the second position, the patient's skin may resiliently move towards the needle tip following the needle protection sleeve, thus leading to an easy and nearly pain-free insertion of the needle tip into the patient's skin.

In some embodiments, the needle protection sleeve may be at least partially received radially inside the rotating sleeve, and the rotating sleeve and the sidewall delimiting the recess defined by the base may be in a thread-like engagement with each other. Preferably, the thread-like engagement of the protection sleeve with the rotating sleeve exhibits a handedness that is opposite to the handedness of the thread-like engagement of the rotating sleeve with the side wall that delimits the recess.

More specifically, the side wall that delimits the recess may comprise an internal thread (i.e., a helical groove), and the rotating sleeve may comprise at least one external thread portion (i.e., a helical rib) configured to engage with said internal thread of the recess. In particular, the internal thread of the recess may be a triple-start thread and the rotating sleeve may comprise three external thread portions, to ensure good stability against tilting movements of the rotating sleeve even if the thread portions are short (e.g., less than 120° in a circumferential direction). Alternatively, the rotating sleeve may have an outer surface comprising a helical groove extending helically about the central axis, and the recess may comprise a thread portion (helical rib) protruding radially inwards towards the central axis, the thread portion being in engagement with the helical groove on the outer surface of the rotating sleeve.

In other embodiments, the rotating sleeve may be at least partially received radially inside the needle protection sleeve. In such embodiments, it is advantageous if the base comprises a central guide element, preferably configured as a hollow cylindrical element, arranged within the recess, the central guide element being extending distally from the top wall into the recess along the central axis. The central guide element may be at least partially received radially inside the rotating sleeve, and the rotating sleeve and the central guide element may be in a thread-like engagement with each other.

In particular, the central guide element may have an outer surface comprising a helical groove extending helically about the central axis, and the rotating sleeve may have at least one thread portion (helical rib) protruding radially inwards towards the central axis configured to engage with the helical groove on the outer surface of the central guide element.

In some embodiments, the rotating sleeve may comprise an internal thread and the needle protection sleeve may comprise at least one external thread portion engaging with said internal thread of the rotating sleeve. In particular, the rotating sleeve may be a triple-start thread and the needle protection sleeve may comprise three external thread portions.

In other embodiments, the needle protection sleeve may comprise an internal thread and the rotating sleeve may comprise at least one external thread portion engaging with said internal thread of the needle protection sleeve. In particular, the internal thread of the needle protection sleeve may be a triple-start thread and the rotating sleeve may comprise three external thread portions.

One of the needle protection sleeve and the rotation lock sleeve may comprise a longitudinal groove extending parallel to the central axis, wherein the other one of the needle protection sleeve and the rotation lock sleeve may comprise a guide cam configured to slide in said longitudinal groove such that the protection sleeve is prevented from rotating with respect to the base when moving from the first position to the second position.

As a resulting effect, the rotation lock sleeve prevents the needle protection sleeve from rotating with respect to the base.

In particular, in some embodiments, the needle protection sleeve may have an outer surface comprising a longitudinal groove, wherein the longitudinal groove extends parallel to the central axis and the rotation lock sleeve may comprise a guide cam being in engagement with said longitudinal groove of the needle protection sleeve. In a preferred version, the needle protection sleeve has an outer surface comprising preferably three longitudinal grooves and the rotation lock sleeve comprises preferably three guide cams, wherein each guide cam is in engagement with one longitudinal groove.

Alternatively, in other embodiments, the rotation lock sleeve may have an inner surface comprising a longitudinal groove, wherein the longitudinal groove extends parallel to the central axis and the needle protection sleeve may comprise a guide cam being in engagement with the longitudinal groove of the rotation lock sleeve. In particular, the inner surface of the rotation lock may comprise at least three longitudinal grooves and the needle protection sleeve may comprise at least three guide cams equidistantly spaced with respect to a circumferential direction to provide sufficient stability.

The patch-like medical device may further comprise a fixation element that is fixedly attached to the base and is in engagement with the rotation lock sleeve such that the rotation lock sleeve is prevented from rotating relative to the base, wherein the fixation element is preferably configured as a fixation ring.

The fixation element may comprise a notch, and the rotation lock sleeve may have an outer surface comprising a ridge extending parallel to the movement axis and being in engagement with the notch of the fixation ring. Preferably, the fixation ring comprises three or four notches, ideally equally distributed along a circumference of the fixation ring for better stability, and the rotation lock sleeve accordingly comprises three or four ridges.

Furthermore, the fixation element may act as a stop element against which the rotation lock sleeve may axially abut in distal direction when the needle protection sleeve is in the first position, thus preventing the rotation lock sleeve and the rotating sleeve from disengaging from the base. The fixation element may be a separate piece attached to the base or may be made in one piece with the base. In case the fixation element is a separate piece, it may be attached to the casing by soldering, screwing or gluing.

The patch-like medical device may further comprise a spring configured to drive the helical movement of the rotating sleeve, the spring being pre-loaded when the needle protection sleeve is in the first position, wherein the spring at least partially relaxes when the needle protection sleeve moves from the first position to the second position.

In particular, the spring may be a leg spring or a torsion spring having a first end and a second end, the first end being connected to the rotation lock sleeve and the second end being connected to the rotating sleeve, thereby exerting a torsion force on the rotating sleeve and urging it to carry out the helical movement. The rotation lock sleeve may have a first bore configured to receive the first end of the spring and the rotating sleeve may have a second bore configured to receive the second end of the spring.

Alternatively, the spring may also be a tension spring. In particular, a tension force may be exerted on the rotating sleeve by the tension spring, pulling the rotating sleeve towards the base, i.e. in proximal direction, thereby urging it to carry out the helical movement, for example by forcing it to follow the helical grooves on the outer surface of the central guide element in embodiments that comprise said central guide element. In such a case, the thread-like engagement of the rotating sleeve and the central guide element preferably has a thread pitch that is sufficiently steep such that said force is sufficient to cause the helical movement. In some embodiments, the tension spring may have a first end being tightly wrapped around a distal protrusion of the base, wherein said distal protrusion is preferably arranged radially inside the cylindrical element and is preferably extending distally along the central axis towards a central opening of the rotating sleeve, and a second end being tightly wrapped around a protrusion of the rotating sleeve, wherein the protrusion of the rotating sleeve is preferably extending proximally along the central axis towards the protrusion of the base.

The helical movement of the rotating sleeve may alternatively also be driven by a force applied to the needle protection sleeve in proximal direction towards the base. In particular, said force may be exerted by the patient's skin. In such a case, the thread-like engagement of the needle protection sleeve and the rotating sleeve preferably has a thread pitch that is sufficiently steep such that said force is sufficient to cause the helical movement.

The patch-like medical device may further comprise a locking mechanism having a locked state in which the locking mechanism prevents the rotating sleeve from moving when the needle protection sleeve is in the first position, and an unlocked state in which the locking mechanism allows the rotating sleeve to carry out the helical movement causing the needle protection sleeve to move from the first position to the second position.

Furthermore, the patch-like medical device may comprise a trigger mechanism operable by the patient, wherein the trigger mechanism is configured to switch the locking mechanism from the locked state to the unlocked state.

The locking mechanism may comprise a latch pivotably connected to the base, the latch being engaged with the rotating sleeve in the locked state to prevent the rotating sleeve from moving and disengaged from the rotating sleeve in the unlocked state to allow the rotating sleeve to carry out the helical movement. In particular, the latch may comprise a tip which engages with an indentation located in a rim of the rotating sleeve. The latch may further comprise a shaft extending through the base parallel to the central axis to reach the side of the base opposite to the contact surface.

Furthermore, the locking mechanism may comprise a lever element being connected to the latch, preferably to the shaft of the latch on the side of the base opposite to the contact surface, for facilitating control of the latch, wherein the lever element is preferably movable between a first lever element position in which the lever element prevents the latch from disengaging from the rotating sleeve, and a second lever element position in which the lever element allows the latch to disengage from the rotating sleeve. The lever element may be shaped in a variety of different ways. In some embodiments, in may be an injection molded part. In other embodiments, it may be made of a sheet metal piece, which may be resiliently bendable. The lever element may have an opening on a first end, in which the end of the shaft of the latch may be fixed. It may have a second end which is, in the first lever element position, biased in distal direction towards the base, in particular bent to abut against a stopper element, the stopper element being attached to the base. In said first lever element position, which corresponds to the locked state of the locking mechanism, the lever element may prevent the latch from disengaging from the rotating sleeve by preventing rotation of the shaft, which is fixedly connected to the first end of the lever element. In the second lever element position, the lever element preferably allows the latch to disengage from the rotating sleeve.

In addition, the locking mechanism may comprise a clamp element configured for clamping the lever element to prevent inadvertent motion of the lever element and the latch, wherein the clamp element is preferably movable between a first clamp position in which the clamp element fixes the lever element with respect to the base in the first lever element position, and a second clamp position in which the clamp element releases the lever element and allows the lever element to move from the first lever element position to the second lever element position. With the lever element moving from the first lever element position to the second lever element position, the latch may be able to pivot and thus to disengage from the rotating sleeve. The rotating sleeve may then be free to perform the helical movement. In cases where a spring is driving said helical movement, the spring is preferably pre-loaded such that the rotating sleeve exerts a force on the latch when the needle protection sleeve is in the first position and the locking mechanism is in the locked state. This force may urge the latch to pivot radially outwards and release the rotating sleeve, but clamping of the lever element in the first lever element position by the clamp element may prevent the latch from pivoting.

In case the fixation element is a fixation ring, the latter preferably covers the latch, such that that latch is hidden and is not in contact with the patient's skin when the patch-like medical device is worn by the patient. In particular, the contact surface may have a recessed area in which the latch may be arranged. The recessed area is then preferably configured to receive the fixation ring such that the fixation ring is flush with the contact surface and covers the latch when attached to the base. In case the fixation element is made in one piece with the base, the latch is preferably hidden within the base.

The trigger mechanism may comprise control circuitry and an actuation mechanism configured to mechanically actuate the clamp element upon receiving an electrical signal from the control circuitry, causing the clamp element to move from the first clamp position to the second clamp position so as to release the lever element. The actuation mechanism may comprise a motor-driven threaded shaft, which is preferably configured to rotate upon receiving a signal from the control circuitry, and a transmission piece that may comprise a fork-like part, wherein the threaded shaft is preferably in a gear-like engagement with the transmission piece. The actuation mechanism may further comprise a pivot arm having a spherically shaped top end that is embraced by the fork-like part of the transmission piece. Upon receiving a signal from the control circuitry, the threaded shaft may start to rotate, thereby moving the transmission piece from a first position to a second position. Preferably, the fork-like part of the transmission piece thereby drags along the spherically shaped top end of the pivot arm, causing the pivot arm to pivot from a first pivot position to a second pivot position. The actuation mechanism may further comprise a wire arrangement which may be at least partially wrapped around the sidewall delimiting the recess and which is preferably configured to urge the clamp element to move in proximal direction from the first clamp position to the second clamp position. The pivot arm may have a nose-like protrusion, which in the first pivot position pushes the clamp element in distal direction (and thereby prevents it from moving) against a force exerted on the clamp element by the resilient wire arrangement. When the pivot arm is being moved to the second pivot position, the clamp element preferably moves in proximal direction, being preferably linearly guided by a guiding structure that may be arranged on the base. As the clamp element moves in proximal direction, it preferably releases the lever element, which then may pivot from the first lever element position to the second lever element position as the latch is pushed radially outward by the rotating sleeve that is urged to rotate by the pre-loaded spring.

In order to activate the trigger mechanism, a button, in particular at least two buttons, preferably three buttons, may have to be pushed simultaneously by a user. The control circuitry may be configured to determine a switching state of the buttons and to output the electrical signal based on said switching state. Having to push several buttons simultaneously may help to reduce the risk of an unwanted accidental activation. The trigger mechanism may further be connected to the pump mechanism and configured to initiate a flow of the liquid substance from the reservoir through the needle to inject the liquid substance into the patient's body once the needle protection sleeve is in the second position. The flow of the liquid substance may be initiated automatically after the needle protection sleeve has reached the second position. Alternatively, initiating the flow of the liquid substance may require the user to push the same buttons again or may require the user to push at least one additional button separately configured to trigger the pump mechanism.

The patch-like medical device may be wirelessly connectable to a remote control device, in particular a tablet computer and/or smartphone, to program and control the patch-like medical device, in particular to set parameters related to the delivery of the liquid substance to the patient. For this purpose, the patch-like medical device may comprise an electronic assembly which comprises a communication module, preferably configured to communicate to the remote control device via Bluetooth.

In some embodiments, the patch-like medical device may comprise one or more light indicators arranged on the patch-like medical device, in particular on the housing upper part, which light indicator may be configured to light up in order to provide visual feedback to the patient to inform the patient about a status of the patch-like medical device, for instance about a pairing status with the remote control device, and/or an energy/power status, and/or a coupling status between the housing upper part and the base in embodiments where the housing upper part and the base are separable, and/or an occlusion event, i.e. when the delivery of the liquid substance may be unexpectedly impaired or interrupted, and/or a filling status of the reservoir, and/or a general malfunction of the patch-like medical device.

The one or more light indicators may be designed as alert symbols, which are easily recognizable by the patient, such as for instance a battery symbol, a general warning sign or sign signalizing wireless communication. The one or more light indicators may also comprise a light ring, wherein the light ring may be arranged around one of the buttons, in particular a top button arranged on top of the housing upper part. The light indicators may be configured to emit light in different colors and with different blinking patterns depending on a status of the patch-like medical device. Light for the one or more alert symbols and/or the one or more light indicators may be provided by light-emitting diodes (LEDs).

Alternatively or additionally to visual feedback, the patch-like medical device may also be configured to emit audible feedback, in particular, it may comprise an acoustic element configured to emit a series of tones at a variety of different acoustic frequencies, to facilitate handling for patients who have impaired vision. Alternatively or additionally, the patch-like medical device may also be configured to emit vibrational feedback, in particular, it may comprise a vibrational element configured to generate vibrations which may be transmitted to the patient's skin via the contact surface. The acoustic element and the vibrational element may be a combined element or may be separate elements.

The patch-like medical device may comprise at least one power source, in particular a battery for driving the pump mechanism and/or powering the control circuitry. In some embodiments, the patch-like medical device comprises a primary battery and a secondary battery, wherein the primary battery is preferably a rechargeable battery, which may be non-removable, and the secondary battery may be a non-rechargeable battery. The secondary battery may in particular be replaceable, i.e. easily removable from the patch-like medical device, and may be used to recharge the primary battery. The secondary battery may have a higher energy density than the primary battery, in particular, the secondary battery may be a Zinc-air battery and the primary battery may be a lithium-polymer (LiPo) battery.

The electronic assembly may comprise a microcontroller, which may be configured to control the visual feedback and/or audible feedback and/or vibrational feedback described above, and/or which may be configured to drive the pump mechanism, and/or which may be part of the control the control circuitry which sends electrical signals to the actuation mechanism.

The pump mechanism may comprise a drive mechanism in form of a motor, in particular a Lavet-type stepping motor. In such a case, the microcontroller may be configured to control a generation of drive voltage pulses which are provided to the motor.

In order to determine a filling state of the reservoir, the patch-like medical device may further comprise a capacitive level sensor assembly. The capacitive level sensor assembly may have three measurement regions, each having pre-determined geometrical dimensions: a liquid measurement region, which corresponds to a region of the reservoir close to an exit end of the reservoir and which is ideally always filled with the liquid substance to be administered to the patient except during an end phase of administration when the piston enters the liquid measurement region to reach the exit end of the reservoir, an environment measurement region, which corresponds to a region of the reservoir located on a second end of the reservoir opposite to the exit end and which should ideally always be free of the liquid substance to be administered to the patient, and a level measurement region, which is located between the liquid measurement region and the level measurement region.

In each of the three measurement regions, a pair of co-planar capacitor plates may be arranged in a capacitor plane adjacent to the reservoir to measure a capacitance value in said measurement region. The reservoir may have a toroidal shape, in which case the capacitor plane be arranged tangentially with respect to the reservoir. A filling level of the reservoir may be calculated taking into account the capacitance values measured in each of the three measurement regions. Additional correction terms may be included when calculating the filing level of the reservoir to take into account that in practice, the liquid measurement region may start to get depleted towards the end phase of administration and that the environment measurement region may be partially filled with the liquid substance when the reservoir is completely full or close to completely full. Such a measurement scheme using three measurement regions may allow for interferences, such as due to external electric fields, to be removed, as long as the interferences are sensed in all three measurement regions proportionally to their respective geometrical dimensions.

Ensuring proper delivery of the liquid substance to the patient may be important to prevent unintentional under- or overdosing. Thus, it may be useful to be able to detect occlusion events, i.e. obstructions in the fluidic path that may occur between the reservoir and the needle tip or at the needle tip.

Different methods of determining an occlusion probability may be employed, in particular, measurements carried out on the pump mechanism, in particular the stepping motor, may be combined with measurements carried out using the capacitive level sensor assembly.

In particular, several input probabilities resulting from different methods may be computed and a combined probability may be computed by OR-ring (i.e. using a logic "OR") such that the output is the probability that none of the input probabilities would declare occlusion.

In a final step, the combined probability may then be compared to a pre-determined critical probability threshold to determine whether an occlusion warning signal is to be emitted or not.

Examples of such methods include a level-estimation method, a lost-step detection method, a motor-torque method, and a rotor vibration detection method.

The level-estimation method may comprise:
a. measuring an absolute change in a filling level based on a capacitance value measured in the level measurement region, a capacitance value measured in the environment region, and a capacitance value measured in the liquid measurement region during a pre-determined number of steps of the stepping motor;
b. computing a change of the filling level per step of the stepping motor based on the absolute change and the pre-determined number of steps;
c. computing a level-method-based probability of occlusion based on a comparison of the computed average change per step of the stepping motor with a first reference change value corresponding to a non-occluded state, and with a second reference change value corresponding to an occluded state.

The change of the filling level per step of the stepping motor may be computed as a mean or medial value over the pre-determined number of steps.

Lost motor steps may be a strong indication of occlusion or other malfunctions. Thus the lost-step-detection method may comprise:
a. measuring a coil flux of the Lavet-type motor;
b. comparing the measured coil flux with a flux threshold value, the flux threshold value being dependent on motor parameters;
c. determining a lost-step-based probability of occlusion based on a comparison of the measured coil flux with the flux threshold value, in particular setting the probability of occlusion to one if the measured coil flux is below the flux threshold value for at least a pre-determined number of motor steps among a total pre-determined number motor steps, in particular wherein the pre-determined number of motor steps is 4 and the total pre-determined number of motor steps is 100.

Due to a certain "elasticity" of the pump mechanism, the detection of occlusion events using the lost-step-detection method may however be delayed compared to other methods.

An estimation of a torque value of the motor based on internal energy may also be used. The motor-torque method may comprise:
a. obtaining a motor response signal over a pre-determined period of time;
b. determining internal energy data based on the motor response signal as a function of time;
optionally: c. applying a noise filter to the internal energy data;
d. detecting a rise in the internal energy data;
e. computing a motor-torque-based probability of occlusion, the motor-torque-based probability of occlusion being proportional to a steepness of the rise in the internal energy data.

Furthermore, a rotor vibration detection method may be used, which is based on evaluating internal energy data of the motor response signal during a passive phase of the motor, in particular evaluating vibrations, i.e. back-and forth movements of the rotor part after an actively driven part of a motor step. Strong dampening of the vibrations occurring in the passive phase may be an indication that the torque of the motor may be approaching its limits, which may point towards an occlusion event.

The rotor vibration detection method may comprise:
a. obtaining a motor response signal during a pre-determined time interval after an actively driven part of a motor step has been completed;
b. determining internal energy data based on the motor response signal during the pre-determined time interval after the motor step has been completed;
c. detecting a change in the internal energy data in the pre-determined time interval after an actively driven part of a motor step has been completed;
d. computing a vibration-based probability of occlusion, the vibration-based probability of occlusion being based on the detected change in the internal energy data.

The motor response signal may be derivable from a voltage and/or current response measured in response to the drive voltage pulses provided to the motor.

It is of course conceivable to select and/or arbitrarily combine any of the four methods disclosed here or to add additional methods to determine the combined probability value.

In case the patch-like medical device has a disposable first device part and a reusable second device part as described above, all components requiring or providing electrical power, in particular the motor, the capacitive level sensor assembly, the batteries and the electronic assembly, are preferably arranged in the reusable second device part to facilitate waste disposal of the disposable first device part and save costs.

In a second aspect, a patch-like medical device with a base and a housing upper part is disclosed, wherein the base and the housing upper part are both securely attachable to each other, as well as easily and quickly separable from each other.

A patch-like medical device is thus provided comprising a housing coupling mechanism for establishing a releasable coupling between the base and the housing upper part, the housing coupling mechanism comprising
a resilient retaining structure arranged on the base, the resilient retaining structure being movable between a coupling position, in which the resilient retaining structure at least partially engages with a coupling recess formed in the housing upper part to lock the housing upper part to the base when the housing upper part is assembled with the base, and a de-coupling position, in which the resilient retaining structure is not engaged with the coupling recess of the housing upper part so as to allow the base and the housing upper part to be separated from one another, and
a clamp element arranged on the base, the clamp element being movable between a clamp hold position, in which the clamp element prevents the resilient retaining structure from moving from the coupling position to the de-coupling position, and a clamp release position, in which the clamp element at least partially releases the resilient retaining structure, allowing the resilient retaining structure to move from the coupling position to the de-coupling position.

Since the clamp element is movable between two defined positions, i.e. the clamp hold position and the clamp release position, a user of the patch-like medical device is provided with a possibility to selectively choose whether the base should be securely attached to the housing upper part to prevent unintentional separation of the housing upper part and the base or whether the base and the housing upper part should be in a state where they are easily separable, for instance to access an interior of the base and/or the housing upper part.

Preferably, the patch-like medical device may be the patch-like medical device of any of the embodiments of the first aspect of the present invention described above, i.e. comprising a needle and a needle protection mechanism, wherein the needle protection mechanism is movable between a protection state in which the needle tip is prevented from piercing the patient's skin, and an insertion state exposing the needle tip to enable piercing the patient's skin.

In an embodiment of the patch-like medical device having a disposable first device part comprising the base forming the housing lower part, and a reusable second device part comprising the housing upper part, being able to quickly and easily release the housing upper part from the base, in particular without damaging the housing upper part, is particularly desirable to enable the patient to quickly exchange a used disposable first device part for a fresh first device part while keeping the same second device part.

In particular in case the base is intended to be disposable and the housing upper part is intended to be reusable, it is especially advantageous for reasons of mechanical reliability and longevity of the reusable housing upper part to have the coupling recess, which is a passive (i.e. non-moveable) feature, being arranged in the reusable housing upper part, and to have the resilient retaining structure, which is moveable and thus potentially mechanically more delicate than the passive coupling recess, being arranged on the disposable base.

In an embodiment in which the needle protection mechanism comprises a locking mechanism having a locked state as described above, in which the locking mechanism prevents the needle protection mechanism from moving from the protection state to the insertion state, and an unlocked state in which the locking mechanism allows the needle protection mechanism to move from the protection state to the insertion state, the clamp element being part of the locking mechanism as described above may be the same element as the clamp element of the housing coupling mechanism, i.e. the housing coupling mechanism and the locking mechanism of the needle protection mechanism may share the clamp element, which enables a particularly compact mechanical design.

The clamp element may be movable in a direction normal to the contact surface of the base between the clamp hold position and the clamp release position. In particular, the clamp element may be closer to the contact surface of the base in the clamp hold position than in the clamp release position.

In case the housing coupling mechanism and the locking mechanism share the same clamp element, the first clamp position, in which the clamp element locks the locking mechanism in the locked state as described in the context of the needle protection mechanism, preferably corresponds to the clamp hold position of the housing coupling mechanism.

Furthermore, the second clamp position, in which the clamp element unlocks the locking mechanism so as to assume the unlocked state, is preferably an intermediate position between the first clamp position and the clamp release position. Preferably, the resilient retaining structure of the housing coupling mechanism is in the coupling position when the clamp element is in the second clamp position.

In some embodiments, the resilient retaining structure may comprise an open retaining ring having a resiliently variable diameter, the open retaining ring being arranged on a side of the base that faces away from the contact surface. The term "open retaining ring" refers here to a ring featuring a gap along its circumference. To vary the diameter of the open retaining ring, the gap may be enlarged or reduced. The coupling recess may be a radial groove formed in the housing upper part, wherein the groove may run along at least a portion of a circumference of the housing upper part, wherein the open retaining ring radially reaches into the radial groove in the coupling position when the housing upper part is placed on the base, and wherein the open retaining ring does not reach into the radial groove in the de-coupling position.

The radial groove may be formed in a circumferentially extending wall of the housing upper part, may extend radially and may be open towards an inside of the housing upper part. In such a case, the diameter of the open retaining ring is preferably larger in the coupling position than in the de-coupling position.

The housing coupling mechanism may further comprise a sealing ring, the sealing ring being arranged on the base to enable watertight coupling between the base and the housing upper part.

The resilient retaining structure may further comprise a resilient wire arrangement forming a first wire arm being connected to a first end of the open retaining ring, and forming a second wire arm being connected to a second end of the open retaining ring. In a preferred embodiment, the resilient wire arrangement corresponds to the wire arrangement described in the context of the needle protection mechanism of the first aspect of the invention, i.e. the needle protection mechanism and the housing coupling mechanism share the resilient wire arrangement.

The base may comprise a base plate, which may be a substantially round disk providing the contact surface to be directed towards the patient's skin on a first side. On a second side of the base plate opposite to the first side, the base may comprises a base wall extending perpendicularly to a surface normal of the base plate and running circumferentially, thereby delimiting an inside of the base in a radial direction.

A base wall groove may be formed along at least a portion of the base wall in an outer surface of the base wall facing radially outwards. The open retaining ring may be arranged partially within the base wall groove to be held in place. In such a case, the sealing ring is preferably arranged between the base plate and the base wall groove.

The base wall may have two breakthroughs through which the open retaining ring may be arranged to pass such that the first and the second ends of the open retaining ring, as well as preferably the first and second spring arms are located in the inside of the base.

Preferably, the resilient wire arrangement acts as a torsion spring and is biased to move the first and the second ends of the open retaining ring towards each other,
wherein in the clamp hold position, the clamp element is arranged between the first and second wire arms to prevent movement of the first and second ends of the open retaining ring towards each other, and
wherein in the clamp release position, the clamp element allows the first and second ends of the open retaining ring to be closer to each other than when the clamp element is in the clamp hold position.

The open retaining ring itself may have a first spring arm and a second spring arm that are configured to provide a counter-bias, i.e. to provide a spring force which pushes the first end and the second end of the open retaining ring away from each other.

To assemble the housing upper part and the base, the housing upper part may be pushed onto the base, which may initially causes the resilient open retaining ring to be pushed radially inwards into the base wall groove. When the housing upper part is pusher further onto the base, the open retaining ring may snap radially outwards into the coupling recess due to the spring force provided via the first and second spring arms of the open retaining ring. Once the housing upper part and the base are assembled, the housing upper part preferably completely covers the open retaining ring, i.e. such that the latter is not visible anymore and protected from tampering.

Preferably, the cylindrical side wall delimiting the recess formed by the base on the side that faces away from the contact surface is arranged concentrically with respect to the open retaining ring, and the cylindrical side wall has an outer diameter that is smaller than than a minimal diameter assumed by the retaining ring. To enable a compact mechanical design of the patch-like medical device, the resilient wire arrangement may be partially wrapped around the cylindrical side wall of the recess.

In a third aspect, a two-part patch-like medical device is the disclosed where the pump mechanism causing the liquid substance to be pumped from the reservoir through the needle is split between the first device part and the second device part, but wherein the two-part patch-like is easy and quick to mechanically assemble.

A patch-like medical device is thus provided comprising a first part, which comprises the reservoir configured to contain the liquid substance and a piston moveably arranged within the reservoir to push the liquid substance out of the reservoir, and comprising a second device part configured to be releasably coupled to the first device part, the second device part comprising a drive mechanism. The first device part comprises a drive train comprising a rotatable sleeve part and a rotatable plug part. The sleeve part and the plug part are engaged with each other in a final engagement state when the first device part and the second device part are coupled and are able to rotate in unison about a common rotation axis in the final engagement state to transmit the mechanical movement from the drive mechanism to the piston to move the piston within the reservoir. The sleeve part and the plug part are separate from each other when the first device part and the second device part are not coupled. To enable quick and easy alignment, the sleeve part and the plug part are configured to forcibly rotate with respect to each other around the common rotation axis until the final engagement state is reached when the first device part and the second device part are brought into coupling in a direction parallel to the common rotation axis.

In some embodiments, the plug part is arranged in the first device part and the sleeve part is arranged in the second device part. However, the opposite is also conceivable.

The drive train may comprise a piston gear system being arranged in the first device part and being connected to the piston and the drive train may comprise a transmission gear system being arranged in the second device part and being connected to the drive mechanism. In such a case, the plug part may comprise a plug gear wheel being in meshing engagement with the piston gear system and the sleeve part may comprise a sleeve gear wheel being in meshing engagement with the transmission gear system.

The transmission gear system may comprise a first transmission gear wheel and a second transmission gear wheel. The piston gear system may comprise a first piston gear wheel, a second piston gear wheel and a third piston gear wheel. To enable a particularly spatially compact drive train, the first and second transmission gear wheels as well as the first, second, and third piston gear wheels may be configured as double-gear wheels, wherein the term "double-gear wheel" defines a set of two gear wheels stacked concentrically on top of each other, wherein the two gear wheels have different diameters, but are attached to each other to rotate in unison.

The first transmission gear wheel may be in meshing engagement with the rotor gear wheel to pick up the rotation movement of the rotor part. The second transmission gear wheel may be in meshing engagement with the first transmission gear wheel and simultaneously in meshing engagement with the sleeve gear wheel to transmit the movement from the first gear wheel to the sleeve part. When the sleeve part and the plug part are in their final engagement state, the sleeve part entrains the plug part to rotate in unison. The plug gear wheel of the plug part may be in meshing engagement with the first piston gear wheel. The second piston gear wheel may be in meshing engagement with both the first piston gear wheel and the third piston gear wheel to transmit the movement from the first piston gear wheel to the third piston gear wheel. The third piston gear wheel may be in meshing engagement with an internal gearing of the piston to move the piston.

The sleeve part may be cylindrically shaped, may extend along the common rotation axis and may have an inner surface comprising an alignment groove extending helically about the common rotation axis.

The plug part may comprise an alignment arm having a lug configured to slide in the alignment groove when the first device part and the second device part are brought into coupling, the lug being guided helically about the common rotation axis along the alignment groove causing the plug part and the sleeve part to rotate with respect to each other until the final engagement state is reached.

The lug may be arranged at a free end of the alignment arm, the free end of the alignment arm entering into engagement with the sleeve part first when the first device part and the second device part are brought into coupling. Preferably, the free end of the alignment arm is resiliently bendable in a radial direction with respect to the common rotation axis such that the lug of the alignment arm and the alignment groove form a snap-fit connection when the plug part and the sleeve part are pushed into engagement with each other parallel to the common rotation axis.

The alignment arm may have a fixed end that is attached to a star-shaped element, the star-shaped element having a plurality of radial arms extending radially in a plane perpendicular to the common rotation axis. The sleeve part may comprise at least one tooth-like protrusion arranged at a first end of the sleeve part, the first end of the sleeve part entering into engagement with the plug part first when the first device part and the second device part are brought into coupling, the at least one tooth-like protrusion extending in a direction parallel to the common rotation axis and being arranged in a tooth gap between two of the radial arms of the star-shaped element when the plug part and the sleeve part are in the final engagement state.

Preferably, the sleeve part has a number of tooth-like protrusions which corresponds to a number of tooth-gaps of the star-shaped element, such that each tooth-like protrusion is located in one tooth-gap when the plug part and the sleeve part are in the final engagement state.

In a particular example, the sleeve part has six tooth-like protrusions and the star-shaped element has six tooth-gaps and the sleeve part comprises six alignment grooves extending helically about the common rotation axis, the six alignment grooves being evenly distributed along a circumference of the inner surface of the sleeve part, and the plug part comprises three alignment arms each having a lug. The lugs are then preferably located in every second alignment groove along the circumference of the inner surface of the sleeve part, when the plug part and the sleeve part are in the final engagement state.

In a fourth aspect, a solution for venting an interior of a patch-like medical device is disclosed with a reusable and a disposable part, while minimizing the risk of dirt impairing the venting.

A patch-like medical device is thus provided comprising:
a first device part and a second device part configured to be releasably coupled to the first device part, the first device part and the second device part defining an interior of the patch-like medical device when coupled together,
wherein the first device part comprises:
   a base having a base plate, the base plate having a contact surface configured to be directed towards a patient's skin, and
   a reservoir configured to contain a liquid substance;
wherein the second device part comprises:
   a housing upper part;
wherein the first device part is intended for one-time-use and the second device part is intended for multiple use, and
wherein the base has a venting arrangement comprising a vent hole extending through the base plate to allow fluids to enter and/or exit the interior of the patch-like medical device, and
wherein the venting arrangement further comprises a vent channel that is formed in the base plate, the vent channel having a first end connected to the vent hole and a second end forming an inlet located on an outer rim of the base plate.

Fluids may be prevented from entering the interior of the patch-like medical device other than via the vent channel and by a sealing ring arranged between the housing upper part and the base.

Having the venting arrangement being arranged in the first device part, which is disposable, i.e. intended for one-time-use, significantly reduces the risk of dirt accumulating in the venting arrangement over time as compared to having the venting arrangement being part of the reusable second device part, which generally has a longer lifetime and is thus more susceptible to an accumulation of dirt. Components providing and/or receiving electrical energy are preferably arranged in the reusable second device part.

Proper venting, as achieved by the venting arrangement of the fourth aspect, is important to prevent an under- or overpressure from occurring in the interior of the patch-like medical device, which could impact the delivery of the liquid substance to the patient, in particular the functioning of the piston within the reservoir. Furthermore, the venting arrangement may also be necessary to ensure the functionality of electronic components, in particular of batteries if they require air to work, such as in the case of Zin-air batteries, and acoustic elements and vibrational elements, which may require air as a transmission medium.

In order to prevent obstruction, the vent hole is preferably arranged in a vent recess formed in the base plate so that a gap remains between the vent hole and the patient's skin when the patch-like medical device is attached to the patient's skin.

The inlet may be arranged on an upper surface of the outer rim of the base plate, the upper surface of the base plate being opposite to the contact surface and thus ideally not covered by the patient's skin.

The inlet may in particular be a perforation extending from the upper surface of the base plate to the contact surface. Alternatively, the inlet may be formed by the vent channel opening out on a lateral circumferential surface of the outer rim of the base plate.

To form a smooth outer surface of the patch-like medical device, the housing upper part may surround and cover the base such that the outer rim of the base plate does not radially protrude beyond the housing upper part when the housing upper part and the base are assembled. In such a case, a small gap however preferably remains between the housing upper part and the upper surface of the outer rim of the base plate in axial direction when the housing upper part and the base are assembled to enable fluids to enter/exit the vent channel between the housing upper part and the base plate.

To prevent damage of sensitive components, in particular of the electronic assembly arranged in the housing upper part, the vent hole is preferably covered by a semi-permeable membrane which allows gases, in particular air, to pass, but which blocks liquids, in particular water.

To secure the patch-like medical device to the patient, a plaster may be attached to the contact surface of the base, the plaster being configured to adhere to the patient's skin. In this case, the vent channel may be formed in the contact surface as a groove being delimited on one side by the plaster, wherein a gap remains between the vent hole and the plaster to allow for proper fluidic circulation.

Preferably, the venting arrangement comprises a plurality of interconnected vent channels each having an end forming an inlet located on an outer rim of the base plate. Such a system of interconnected vent channels may allow for gases, in particular air, to reach the vent hole from inlets disposed all around the outer rim, which at the same time also facilitates getting rid of liquids, in particular water, which may get stuck in the vent channels if the patient wears the patch-like medical device while showering or swimming.

The various aspects may each be implemented individually or combined with each other as desired.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows an exploded view of a patch-like medical device according to a first embodiment of the present invention;
- Fig. 2A: shows a sectional view of the patch-like medical device of Fig. 1 in a first position;
- Fig. 2B: shows a sectional view of the patch-like medical device of Fig. 1 in an intermediate position;
- Fig. 2C: shows a sectional view of the patch-like medical device of Fig. 1 in a second position;
- Fig. 3A: shows a detailed perspective view of a rotating sleeve according to the first embodiment of Fig. 1;
- Fig. 3B: shows a detailed perspective view of a rotation lock sleeve according to the first embodiment of Fig. 1;
- Fig. 3C: shows a detailed perspective view of a needle protection sleeve according to the first embodiment of Fig. 1;
- Fig. 3D: shows a detailed perspective view of fixation ring according to the first embodiment of Fig. 1;
- Fig. 4A: shows a first step of a method of assembly involving components of the first embodiment of Fig. 1;
- Fig. 4B: shows a second step of the method of assembly involving components of the first embodiment of Fig. 1;
- Fig. 4C: shows a third step of the method of assembly involving components of the first embodiment of Fig. 1;
- Fig. 4D: shows the sleeves of the first embodiment of Fig. 1 in a preloaded state, i.e. in the first position, after the third step of the method of assembly;
- Fig. 5: shows an exploded view of a patch-like medical device according to a second embodiment of the present invention;
- Fig. 6: shows a sectional view of the patch-like medical device of Fig. 5 in a first position;
- Fig. 7A: shows a detailed perspective view of a rotating sleeve according to the second embodiment of Fig. 5;
- Fig. 7B: shows a detailed perspective view of a rotation lock sleeve according to the second embodiment of Fig. 5;
- Fig. 7C: shows a detailed perspective view of a needle protection sleeve according to the second embodiment of Fig. 5;
- Fig. 7D: shows a detailed perspective view of fixation ring according to the second embodiment of Fig. 5;
- Fig. 8: illustrates schematically an actuation of the patch-like medical device;
- Fig. 9A: shows a locked state of a first embodiment of a locking mechanism according to the present invention in a perspective view;
- Fig. 9B: shows an unlocked state of the first embodiment of the locking mechanism of Fig. 9A;
- Fig. 9C: shows an enlarged perspective view of a latch and a lever element that are part of the first embodiment of the locking mechanism of Fig. 9A;
- Fig. 10A: shows a top view of the locking mechanism of Fig. 9A in the locked state;
- Fig. 10B: shows a top view of the locking mechanism of Fig. 9A in the unlocked state;
- Fig. 11A: shows a bottom view of the locking mechanism of Fig. 9A in the locked state;
- Fig. 11B: shows a bottom view of the locking mechanism of Fig. 9A in the unlocked state;
- Fig. 12: shows a locked state of a second embodiment of a locking mechanism according to the present invention in a perspective view;
- Fig. 13: shows a locked state of a third embodiment of a locking mechanism according to the present invention in a perspective view;
- Fig. 14: shows a bottom view of a base where the fixation ring is formed by the base itself;
- Fig. 15: shows a perspective view of an embodiment of the patch-like medical according to the present invention having a first device part and a second device part, where the first device part and the second device part are not connected to each other;
- Fig. 16: shows a perspective view of the embodiments of the first device part and the second device part shown in Fig. 15, but where the first device part are coupled to each other;
- Fig. 17: shows an exploded view of the first device part of the embodiment shown in Fig. 15;
- Fig. 18: shows an enlarged exploded view of the reservoir assembly and the needle of the embodiment shown in Fig. 17;
- Fig. 19: shows a top view of the reservoir assembly shown in Fig. 18;
- Fig. 20A: shows a sectional view along the sectional plane A'-A' marked in Fig. 19 when a filling needle is inserted;
- Fig. 20B: shows the sectional view of Fig. 20A without the filling needle being inserted,
- Fig. 21: shows an additional exploded view of the embodiment shown in Fig. 17;
- Fig. 22: shows a perspective view of the open retaining ring;
- Fig. 23A: shows a sectional view of the first device part and the second device part where the housing coupling mechanism is in the coupling position and the housing upper part is locked to the base;
- Fig. 23B: shows an enlarged view of the encircled are marked in Fig. 23A;
- Fig. 23C: shows a sectional view of the first device part and the second device part where the housing coupling mechanism is in the de-coupling position and the housing upper part can be separated from the base;
- Fig. 23D: shows an enlarged view of the encircled are marked in Fig. 23C;
- Fig. 24A: shows a sectional view of the first device part shown in Fig. 15 along a first sectional plane, where the clamp element is in the first position;
- Fig. 24B: shows a sectional view of the first device part shown in Fig. 15 along the first sectional plane, where the clamp element is in the intermediate position/the second position and the sleeves of the needle protection mechanism are retracting;
- Fig. 24C: shows a sectional view of the first device part shown in Fig. 15 along the first sectional plane, where the clamp element is in the intermediate position/the second position and the sleeves of the needle protection mechanism are fully retracted;
- Fig. 24D: shows a sectional view of the first device part shown in Fig. 15 along the first sectional plane, where the clamp element is in the clamp release position and housing coupling mechanism is in the de-coupling state;
- Fig. 25A: shows the state of Fig. 24A along a second sectional plane perpendicular to the first sectional plane;
- Fig. 25B: shows the state of Fig. 24B along the second sectional plane perpendicular to the first sectional plane;
- Fig. 25C: shows the state of Fig. 24C along the second sectional plane perpendicular to the first sectional plane;
- Fig. 25D: shows the state of Fig. 24D along the second sectional plane perpendicular to the first sectional plane;
- Fig. 26A: shows an enlarged portion of a sectional view of the patch-like medical device when the housing upper part and the base are in an assembled state prior to emergency decoupling;
- Fig. 26B: shows an enlarged portion of a sectional view of the patch-like medical device when the housing upper part and the base are in an assembled state after emergency decoupling;
- Fig. 26C: shows a perspective view of a frame structure together with a further embodiment of an actuation mechanism of a patch-like medical device according to the present invention;
- Fig. 26D: shows a top view of the actuation mechanism of Fig. 26C, where the pivot arm is in the first pivot position;
- Fig. 26E: shows a top view of the actuation mechanism of Fig. 26C, where the pivot arm is in the second pivot position;
- Fig. 27: shows an exploded view of the drive mechanism and the drive train of the patch-like medical device of Fig. 15.
- Fig. 28A: shows an exploded view of the stator and the coil of the Lavet-type motor;
- Fig. 28B: shows a perspective view of the stator and the coil of the Lavet-type motor when assembled;
- Fig. 29A: shows a perspective view of the rotatable sleeve part of the drive train of Fig. 27;
- Fig. 29B: shows a perspective view of the rotatable plug part of the drive train of Fig. 27;
- Fig. 29C: shows a top view of the plug part and the sleeve part being in their finale engagement state;
- Fig. 29D: shows a sectional view of the plug part and the sleeve part being in their final engagement state along the sectional plane B'-B' marked in Fig. 29C;
- Fig. 30: shows an exploded view of the base part, the housing upper part and the plaster of the patch-like medical device of Fig. 15;
- Fig. 31: shows a portion of a section view of the base and the housing upper part being in the coupled state illustrating a fluidic venting path of the patch-like medical device of Fig. 15;
- Fig. 32: shows a partially exploded view of components belonging to the second device part of the patch-like medical device of Fig. 15:
- Fig. 33: shows a perspective view of the second device part of the patch-like device of Fig. 15;
- Fig. 34: shows an exploded view of the housing upper part, the frame structure, the electronic circuit board, the capacitive level sensor assembly, the base and the reservoir of the patch-like medical device of Fig. 15;
- Fig. 35A: shows a bottom view of the capacitive level sensor assembly being assembled with the frame structure;
- Fig. 35B: shows a side vie
- Fig. 36: shows a top view of the capacitive level sensor assembly being assembled with the frame structure;
- Fig. 37: schematically illustrates a method for occlusion detection;
- Fig. 38: shows a schematic illustration of the patch-like medical device of Fig. 15 being wirelessly connected to a remote control device, and
- Fig. 39: a perspective view of the housing upper part of the patch-like medical device of Fig. 15 illustrating user interaction features.

### DESCRIPTION OF PREFERRED EMBODIMENTS

### Needle protection mechanism

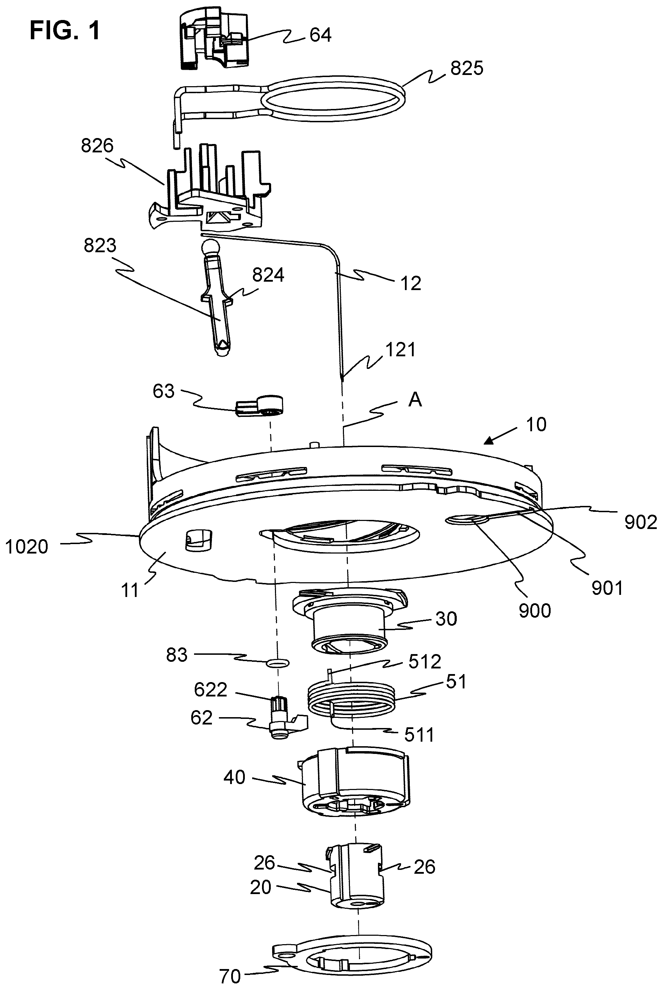

Figures 1 to 4D illustrate a patch-like medical device according to a first embodiment of the present invention.

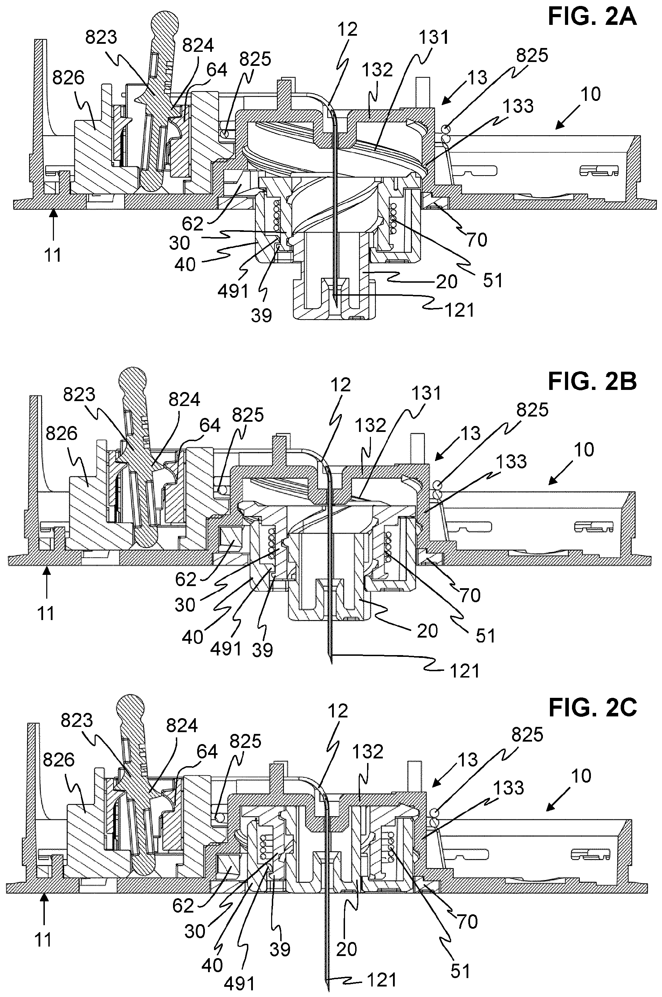

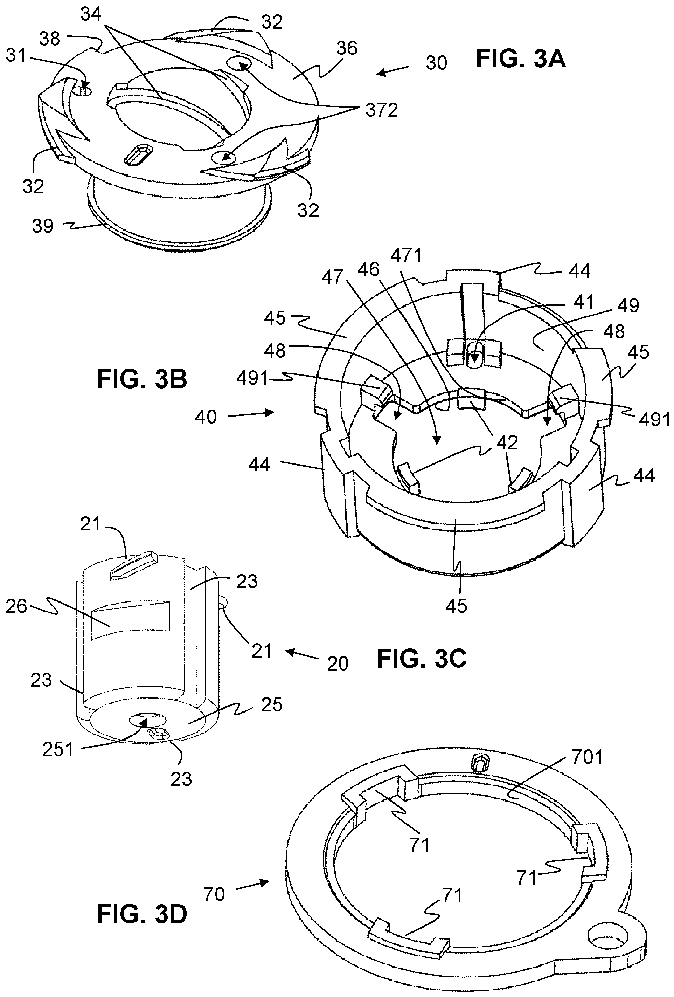

Fig. 1 shows an exploded view comprising individual components, Figs. 2A-C show a sectional view depicting how the individual components are in engagement with each other in this first embodiment of the patch-like medical device, and Figs. 3A-D show perspective detail views of selected individual components. The patch-like medical device comprises a base 10 having a contact surface 11 configured to be directed towards a patient's skin. Said contact surface 11 may comprise an adhesive layer or may be configured to be attached to an adhesive plaster to enable the patch-like medical device to be worn by the patient over an extended period of time. The patch-like medical device further comprises a needle 12 fixedly positioned relative to the base 10. The needle 12 has a needle tip 121 which protrudes from the contact surface 11 and which is configured for piercing the patient's skin. In the embodiments shown here, the needle 12 is a cannula configured to deliver a liquid substance through the patient's skin. The base 10 defines a recess 13 (visible in Fig. 2A-2C) arranged on a side of the base 10 being opposite to the contact surface 11. The recess is delimited in axial direction by a top wall 132. In radial direction, the recess 13 is delimited by a cylindrical side wall 133.

Furthermore, the patch-like medical device comprises a rotating sleeve 30 (shown separately in Fig. 3A) configured to perform a helical movement with respect to the base 10, a rotation lock sleeve 40 (shown separately in Fig. 3B), a needle protection sleeve 20 (shown separately in Fig. 3C) and a fixation element in form of a fixation ring 70 (shown separately in Fig. 3D).

The patch-like medical device shown here further comprises a spring 51. In this first embodiment, the spring 51 is a helical torsion spring capable of exerting a twisting force. The spring 51 has a first end 511 and a second end 512, wherein the rotation lock sleeve 40 has a first bore 41 (as shown in Fig. 3B) in which the first end 511 of the spring is received. The rotating sleeve 30 has a second bore 31 (shown in Fig. 3A) in which the second end 512 of the spring is received.

Prior to use, i.e. prior to piercing the patient's skin, the needle protection sleeve 20 is in a first position, as depicted in Fig. 2A, where it extends beyond the needle tip 121 to protect the latter. In this first position, the rotating sleeve 30 partially protrudes from the contact surface 11 in distal direction. The spring 51 is pre-loaded and in particular exerts a twisting force between the rotating sleeve 30 and the rotation lock sleeve 40. Since the rotation lock sleeve 40 is prevented from rotating by the fixation ring 70, the rotating sleeve 30 is urged to carry out a helical movement in proximal direction. However, to prevent the rotating sleeve 30 from moving prior to use, the rotating sleeve 30 may be held back by a latch 62, which is part of a locking mechanism that will be explained in further detail below. Once the locking mechanism is switched from a locked state to an unlocked state, the latch 62 disengages from the rotating sleeve 30 and allows the latter to rotate due to the twisting force exerted on it by the spring 51. As shown in Fig. 2B, while the spring 51 at least partially relaxes, the rotating sleeve 30 performs the helical movement, i.e. in this case screws itself into the recess 13 while dragging along the rotation lock sleeve 40, which is hooked to a distal rim 39 of the rotating sleeve via three hooks 491. The dragged rotation lock sleeve 40 performs a linear movement in proximal direction, as it is prevented from rotating by the fixation ring 70, which in turn is attached to the base 10. The needle protection sleeve 20, which is prevented from rotating by the rotation lock sleeve 40, linearly move towards the base 10 while effectively getting screwed into the rotating sleeve 30, since the needle protection sleeve 20 is in a thread-like engagement with the rotating sleeve 30. Ultimately, the needle protection sleeve reaches its second position (shown in Fig. 2C), in which the needle tip 121 is exposed. Fig. 2C shows a preferred second position, where all the sleeves are fully retracted inside the recess 13 of the base 10.

The rotating sleeve 30 is in a thread-like engagement with the side wall 133 that delimits the recess 13. The thread-like engagement of the protection sleeve 20 with the rotating sleeve 30 exhibits a handedness that is opposite to the handedness of the thread-like engagement of the rotating sleeve 30 with the side wall 133 that delimits the recess 13. In this first embodiment, said side wall 133 comprises an internal thread 131 in form of a triple-start internal thread (visible in Fig. 2A). The rotating sleeve 30 comprises three external thread portions 32 (shown in detail in Fig. 3A) configured to engage with said triple-start internal thread 131 of the recess 13. The three external thread portions 32 are arranged on a proximal rim 36 of the rotating sleeve 30, the proximal rim 36 extending radially outwards. In the first position, said proximal rim 36 is configured to axially abut in distal direction against rim portions 45 of the rotation lock sleeve 40 (see Fig. 3B), which rim portions 45 also extend radially outwards. The rim portions 45 are in turn configured to axially abut in distal direction against the fixation ring 70 in the first position, which prevents the rotation lock sleeve 40 and hence also the rotating sleeve 30 from disengaging from the patch-like medical device. Two bores 372 are furthermore arranged off-center with respect to the central axis A to enable the insertion of a tool when initially assembling the components, such that the rotating sleeve may be rotated using said tool to thereby pre-tension the spring 51.

In an alternative version of this first embodiment (not shown in the figures), the rotating sleeve 30 may have an outer surface comprising a helical groove extending helically about the central axis A and the recess 13 may comprise a thread portion protruding radially inwards towards the central axis A configured to engage with the helical groove on the outer surface of the rotating sleeve 30.

In this first embodiment, the needle protection sleeve 20 is at least partially received radially inside the rotating sleeve 30, and the needle protection sleeve 20 and the rotating sleeve 30 are in a thread-like engagement with each other. In this particular example, the rotating sleeve 30 comprises an internal thread 34 which is a triple-start internal thread and the needle protection sleeve 20 comprises three external thread portions 21 engaging with said triple-start internal thread 34 of the rotating sleeve 30. As shown in Fig. 3C, the needle protection sleeve 20 has, at a distal end, a protection sleeve front wall 25 defining a protection sleeve contact surface, which is in contact with the patient's skin when the patch-like medical device is applied to the patient. The protection sleeve front wall 25 has a needle hole 251, through which needle hole 251 the needle tip 121 protrudes when the needle protection sleeve 20 is in the second position. The three external thread portions 21 are arranged on an outer surface of the needle protection sleeve 20 at a proximal end of the protection sleeve 20. Furthermore, the outer surface of the needle protection sleeve 20 comprises three longitudinal grooves 23, each longitudinal groove 23 extending parallel to the central axis A from the first end to the second end and being arranged between two of the external thread portions 21 with respect to the circumferential direction. In the embodiment shown here, the three longitudinal grooves 23 have an essentially rectangular cross section, they may however have any other suitable shape. In addition, the outer surface of the needle protection sleeve 20 comprises two indentations 26 extending perpendicularly to the longitudinal grooves and being arranged radially opposite to each other. These indentations 26 are used when initially assembling the components, as will be described below.

The rotation lock sleeve 40 (shown in Fig. 3B) has, at a distal end, a rotation lock sleeve front wall 46, which partially caps the rotation lock sleeve 40. The rotation lock sleeve front wall 46 has an opening 47 centered around the central axis A, through which opening 47 the needle protection sleeve 20 glides when moving from the first position to the second position. To prevent the needle protection sleeve 20 from rotating with respect to the base 10, the rotation lock sleeve 40 comprises three essentially rectangular guide cams 42 protruding radially inward from an edge 471 of the opening 47, wherein each guide cam 42 is in engagement with one of the three longitudinal grooves 23 of the needle protection sleeve 20. Instead of being essentially rectangular, the guide cams 42 may also have any other suitable form. The rotation lock sleeve 40 furthermore comprises three hooks 491 protruding radially inwards from an inner wall 49 of the rotation lock sleeve 40, wherein each hook 491 is arranged between two of the guide cams 42 with respect to the circumferential direction. In this embodiment, the hooks 491 are configured to engage with the distal rim 39 of the rotating sleeve 30 to form a snap-fit connection. In order to facilitate demolding of the hooks 491 during fabrication of the rotation lock sleeve 40, a notch 48 extends radially outwards from the edge 471 of the opening 47 underneath each hook 491. The rim portions 45 described above are arranged at a proximal end of the rotation lock sleeve 40.

Furthermore, the rotation lock sleeve 40 has an outer surface comprising three longitudinal ridges 44 with essentially rectangular profiles, each extending parallel to the central axis A. As shown in Fig. 3D, the fixation ring 70 comprises three notches 71 with essentially rectangular cross-sections extending radially outwards from an inner edge 701 of the fixation ring 70. Each notch 71 is in engagement with one of the ridges 44 of the rotation lock sleeve 40, thereby preventing it from rotating with respect to the base 10. Instead of having essentially rectangular profiles, the longitudinal ridges 44 may have any other suitable profiles, provided that the notches 71 have a corresponding cross-section.

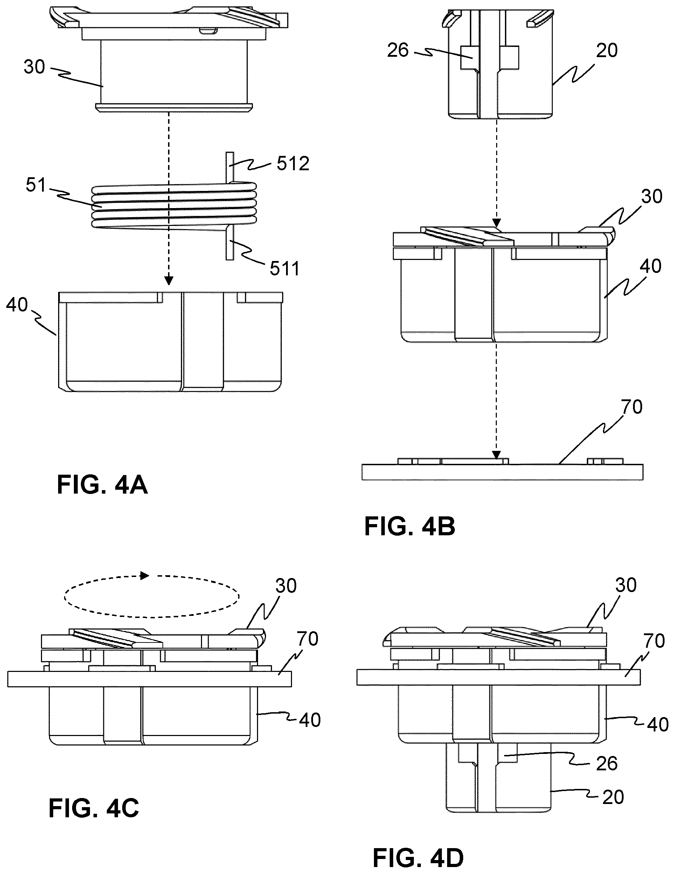

Figs. 4A-4D show how the sleeves of the first embodiment of the present invention may be assembled. In a first step (Fig. 4A), the spring 51 is placed inside the rotation lock sleeve 40 such that first end 511 is received in the first bore 41 (visible in Fig. 3B), and the rotating sleeve 30 is placed inside the rotation lock sleeve 40 such that the second end 512 of the spring 51 is received in the second bore 31 (visible in Fig. 3A). In a second step (Fig. 4B), the needle protection sleeve 20 is inserted into the rotating sleeve 30 from the top, i.e. inserted in distal direction, yielding a pre-assembly comprising the three sleeves and the spring 51, and the pre-assembly is put into the fixation ring 70 from the top. In a third step (Fig. 4C), the rotating sleeve 30 is rotated with respect to the fixation ring 70, which is preferably fixedly mounted in a temporary mount, by using a tool (not shown) inserted in the bores 372 of the rotating sleeve 30 as described above. Rotating the rotating sleeve 30 causes the spring 51 to be pre-loaded and also causes the needle protection sleeve 20 to move in distal direction to finally reach the first position shown in Fig. 4D. Once this first position is reached, the pre-assembly may be kept in this first position by gripping means (not shown) that hold onto the two indentations 26 to prevent the needle protection sleeve 20 from screwing itself back into the rotating sleeve 30 once the tool is removed from the bores 372. The pre-assembly plus the fixation ring 70 may then be mounted into the base 10. The rotating sleeve 30 is then locked by a locking mechanism 60 that will be described in more detail below. Once the rotating sleeve 30 is locked, the gripping means may be removed.

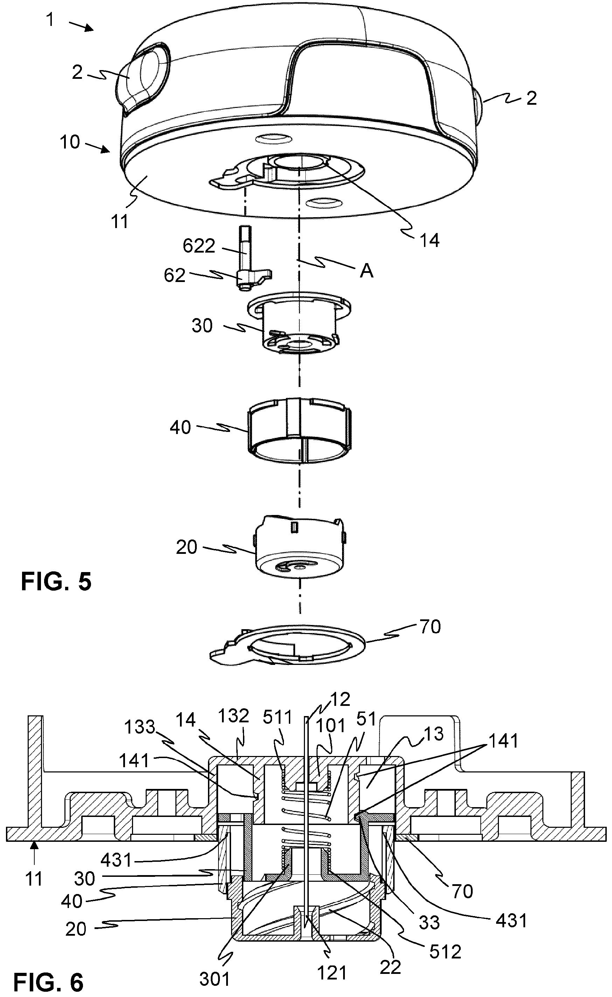

Figs. 5 to 7D illustrate a patch-like medical device according to a second embodiment of the present invention.

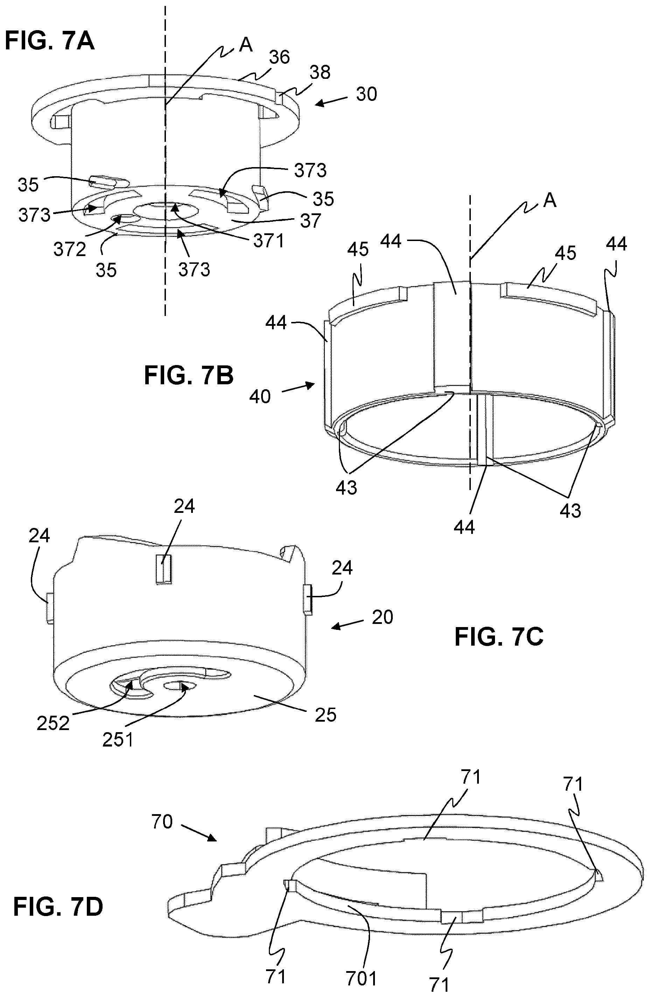

Fig. 5 shows an exploded view comprising individual components, Fig. 6 shows a sectional view depicting how the individual components are in engagement with each other in this second embodiment of the patch-like medical device and Figs. 7A-7D show perspective detail views of selected individual components.

Like the first embodiment described above, the second embodiment comprises a base 10 having a contact surface 11 configured to be applied to a patient's skin, wherein the contact surface 11 may comprise an adhesive layer or may be attached to an adhesive sheet, and a needle (cannula) 12 fixedly positioned relative to the base 10, wherein the needle 12 has a needle tip 121 which protrudes from the contact surface 11 and which is configured for piercing the patient's skin. The base 10 defines a recess 13 arranged on a side of the base 10 being opposite to the contact surface 11, the recess being delimited in axial direction by a top wall 132 and in radial direction by a cylindrical side wall 133.

The patch-like medical device according to the second embodiment comprises a rotating sleeve 30 (shown separately in Fig. 7A), a rotation lock sleeve 40 (shown separately in Fig. 7B), a needle protection sleeve 20 (shown separately in Fig. 7C) and a fixation ring 70 (shown separately in Fig. 7D).

In this second embodiment, the base 10 comprises a central guide element configured as a hollow cylindrical element 14 arranged within the recess 13 and extending in the distal direction along the central axis A, wherein the cylindrical element 14 is at least partially received radially inside the rotating sleeve 30.

In this second embodiment, the spring 51 is a tension spring having a first end 511 being tightly wrapped around a distal protrusion 101 of the base 10 arranged radially inside the cylindrical element 14 and extending distally along the central axis A towards a central opening 371 of the rotating sleeve 30, and a second end 512 being tightly wrapped around a protrusion 301 of the rotating sleeve 30 extending proximally along the central axis A towards the protrusion 101 of the base 10. In the first position (Fig. 6), the spring 51 is pre-loaded and exerts a tension force on the rotating sleeve 30, pulling the rotating sleeve 30 in proximal direction towards the base 10. Similar to the first embodiment described above, once the locking mechanism is switched from a locked state to an unlocked state, the latch 62 disengages from the rotating sleeve 30. The rotating sleeve 30, being pulled by the spring 51, then caries out the helical movement following helical grooves 141 that are arranged on an outer surface of the cylindrical element 14, thereby retracting into the recess 13. The helical movement simultaneously causes the rotating sleeve 30 to screw itself into the needle protection sleeve 20, since the needle protection sleeve 20 is in a thread-like engagement with the rotating sleeve 30 while being prevented from rotating with respect to the base 10, and, in this second embodiment, the rotating sleeve 30 is at least partially received radially inside the needle protection sleeve 20. Here, the thread-like engagement of the protection sleeve 20 with the rotating sleeve 30 exhibits a handedness that is opposite to the handedness of the thread-like engagement between the rotating sleeve 30 and the outer surface of the cylindrical element 14. The needle protection sleeve 20 linearly moves in proximal direction towards the base 10 while being prevented from rotating by longitudinal grooves 43 arranged in an inner surface of the rotation lock sleeve 40 that are in engagement with four guide cams 24 arranged on an outer surface of the needle protection sleeve 20. The needle protection sleeve 20 thereby moves relative to the rotation lock sleeve 40 until it reaches a proximal end of the rotation lock sleeve 40. At the proximal end of the rotation lock sleeve 40, the needle protection sleeve 20 then abuts in proximal direction against end facets 431 of the longitudinal grooves 43, which then causes the needle protection sleeve 20 to drag the rotation lock sleeve 40 along in proximal direction until all sleeves are ideally fully retracted within the recess 13. During retraction, the rotation lock sleeve 40 is furthermore prevented from rotating with respect the base 10 by the fixation ring 70.

In the following, features of this second embodiment are described in more details.

As mentioned above, the rotating sleeve 30 and the cylindrical element 14 are in a thread-like engagement with each other. The cylindrical element 14 has an outer surface comprising three helical grooves 141 (see Fig. 6) extending helically about the central axis A. The rotating sleeve 30 has three thread portions 33 protruding radially inwards towards the central axis A, wherein configured to each thread portion 33 engages with one of the helical grooves 141 on the outer surface of the cylindrical element 14. The rotating sleeve 30 has, at a distal end, a rotating sleeve front wall 37 having a central opening 371, which extends as a protrusion in proximal direction (as visible in the cross-sectional view in Fig. 6) and through which the needle 12 is able to pass. A bore 372 is furthermore arranged off-center with respect to the central axis A to enable the insertion of a tool when initially assembling the components, such that the rotating sleeve may be rotated using said tool to thereby pre-loading the spring 51. In addition, three arched openings 373 are arranged around the central opening 371. In the second position, i.e. when all the sleeves are fully retracted, portions of the cylindrical element 14 comprising the helical grooves 141 are protruding into said arched openings 373 (not directly visible in the figures). This enables the length of the helical grooves 141 to be maximized while keeping a compact configuration. The three thread portions 33 in engagement with the helical grooves 141 are arranged on the inside of the rotating sleeve 30 at a proximal end of the rotating sleeve 30. At a proximal end opposite to the distal end, a rim 36 furthermore extends radially outwards. In the first position, the rim 36 may axially abuts against rim portions 45 of the rotation lock sleeve 40 in the distal direction, which rim portions 45 are arranged at a proximal end of the rotation lock sleeve 40 and also extend radially outwards. The rim portions 45 of the rotation lock sleeve 40 in turn abut axially against the fixation ring 70 in distal direction, which prevents the rotation lock sleeve 40 from disengaging from the base in the first position.

Like in the first embodiment, the needle protection sleeve 20 and the rotating sleeve 30 are in a thread-like engagement with each other. However, in this second embodiment, the rotating sleeve 30 is at least partially received radially inside the needle protection sleeve 20. The needle protection sleeve 20 comprises an internal thread 22 which is a triple-start internal thread and the rotating sleeve 30 comprises three external thread portions 35 engaging with said triple-start internal thread 22 of the needle protection sleeve 20. As shown in Fig. 7C, the needle protection sleeve 20 has, at a distal end, a protection sleeve front wall 25 defining a protection sleeve contact surface, which is in contact with the patient's skin when the patch-like medical device is applied to the patient. Like in the first embodiment, the protection sleeve front wall 25 has a needle hole 251, through which needle hole 251 the needle tip 121 protrudes when the needle protection sleeve 20 is in the second position. Furthermore, in this second embodiment, the protection sleeve front wall 25 has an opening 252 shaped as semi-circle extending around the needle hole 251, which is configured to allow a tool to fit through when initially assembling the components as described above.

As shown in Fig. 7B, the rotation lock sleeve 40 according to the second embodiment has an inner surface comprising four longitudinal grooves 43, wherein the four longitudinal grooves 43 each extend parallel to the central axis A. At a proximal end of the rotation lock sleeve 40, the longitudinal grooves 43 are delimited by end facets 431. Each longitudinal groove 43 is arranged between two of the rim portions 45 with respect to the circumferential direction. The needle protection sleeve 20, as shown in Fig. 7D, comprises four guide cams 24 arranged on an outer surface of the needle protection sleeve 20. Each of the four guide cams 24 is in engagement with one of the longitudinal grooves 43 of the rotation lock sleeve 40. As a resulting effect, the needle protection sleeve 20 is prevented from rotating with respect to the base and moves in a direction parallel to the central axis A from the first position to the second position. Furthermore, during retraction, the guide cams 24 abut against the end facets 431 of the longitudinal grooves 43, which then enables the needle protection sleeve 20 to drag the rotation lock sleeve in proximal direction as described above.

Furthermore, in the second embodiment the rotation lock sleeve 40 has an outer surface comprising four ridges 44 with essentially rectangular profiles, each extending parallel to the central axis A, and wherein each ridge 44 is arranged radially opposite to one of longitudinal grooves 43. As shown in Fig. 7D, the fixation ring 70 comprises four notches 71 with essentially rectangular cross-sections extending radially outwards from an inner edge 701 of the fixation ring 70. Each notch 71 is in engagement with one of the ridges 44 of the rotation lock sleeve 40, thereby linearly guiding the latter and preventing it from rotating with respect to the base 10. Instead of a rectangular shape, the ridges 44 and the corresponding notches 71 may also have any other suitable form that enables an engagement of the notches 71 with the ridges 44.

The patch-like medical device according to both the first embodiment and the second embodiment discussed above further comprises a locking mechanism 60 having a locked state in which the locking mechanism 60 prevents the rotating sleeve 30 from moving when the needle protection sleeve 20 is in the first position, and an unlocked state in which the locking mechanism 60 allows the rotating sleeve 30 to carry out the helical movement causing the needle protection sleeve 20 to move from the first position to the second position.

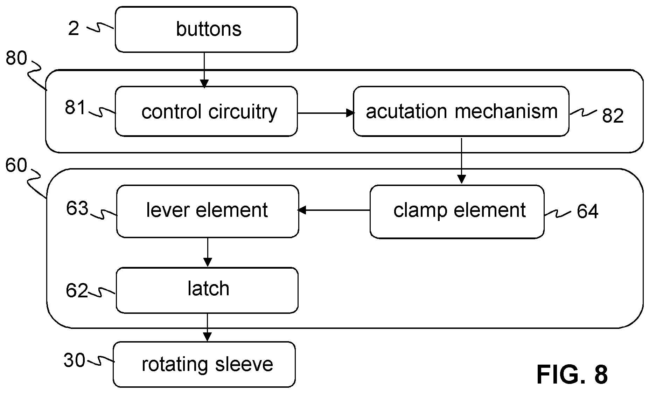

Preferably, the patch-like medical device further comprises a trigger mechanism 80 operable by the patient, for instance by pushing a button, in particular by simultaneously pushing at least two buttons 2 arranged on the patch-like medical device 1 as shown in Fig. 5, preferably by pushing three buttons, wherein the third button is arranged on a top surface of the patch-like medical device 1 (not visible in Fig. 5). Although not explicitly shown in Fig. 1, the patch-like medical device according to the first embodiment also preferably comprises an housing upper part with one or more buttons analogously to the second embodiment shown in Fig. 5. The trigger mechanism 80 is configured to switch the locking mechanism 60 from the locked state to the unlocked state. As schematically illustrated in Fig. 8, the trigger mechanism ideally comprises control circuitry 81 and an actuation mechanism 82. The locking mechanism 60 preferably comprises a latch 62, a lever element 63 connected to the latch 62 and a clamp element 64 configured to clamp the lever element 63. The latch 62 is pivotably connected to the base 10. In the locked state, the latch 62 is engaged with the rotating sleeve 30 to prevent the rotating sleeve 30 from moving. In the unlocked state, the latch 62 is disengaged from the rotating sleeve 30 to allow the rotating sleeve 30 to carry out the helical movement. The actuation mechanism is configured to mechanically actuate a clamp element 64 upon receiving an electrical signal from the control circuitry 81, thereby causing the clamp element 64 to move from a first clamp position to a second clamp position. The clamp element 64 then releases the lever element 63, allowing it to move from a first lever element position to a second lever element position as the latch pivots outwards and disengages from the rotating sleeve 30, the rotating sleeve 30 being now free to perform the helical movement causing the needle protection sleeve 20 to move from the first position to the second position to expose the needle tip 121.

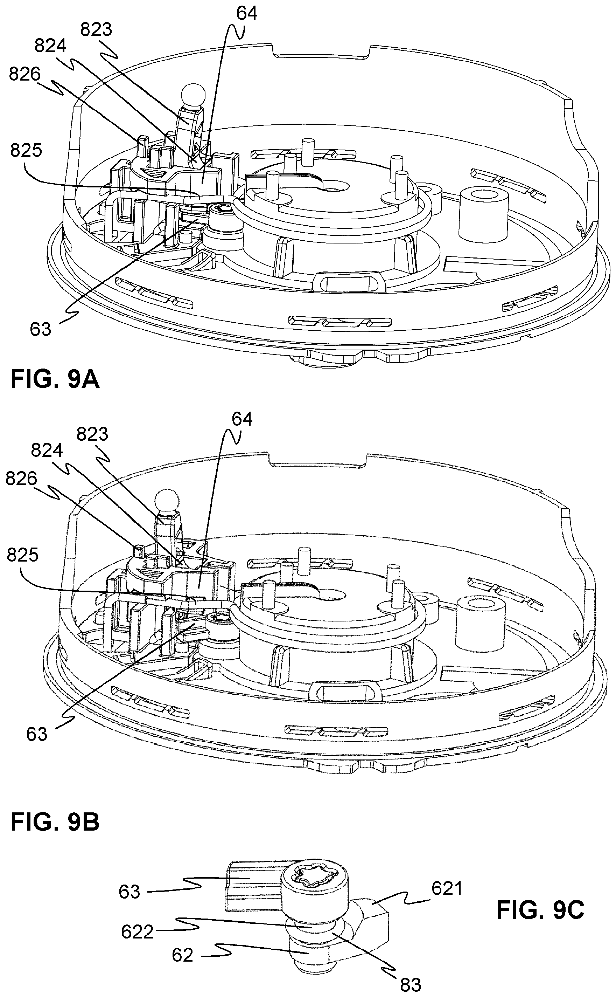

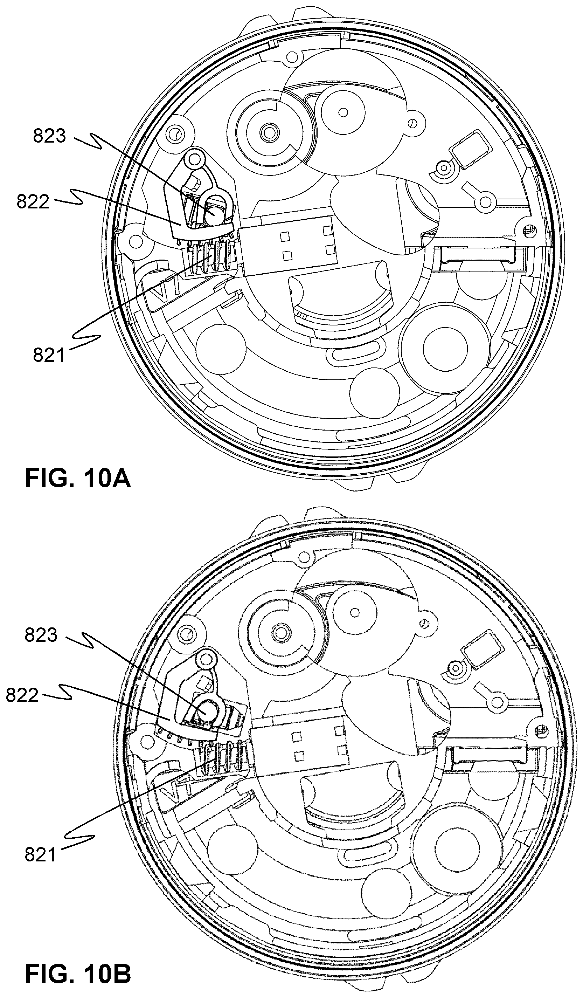

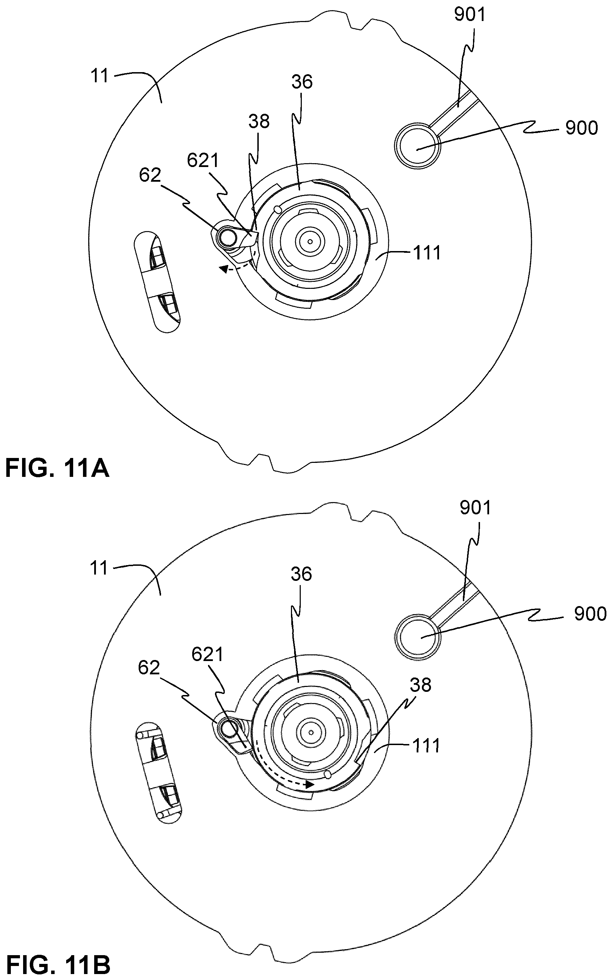

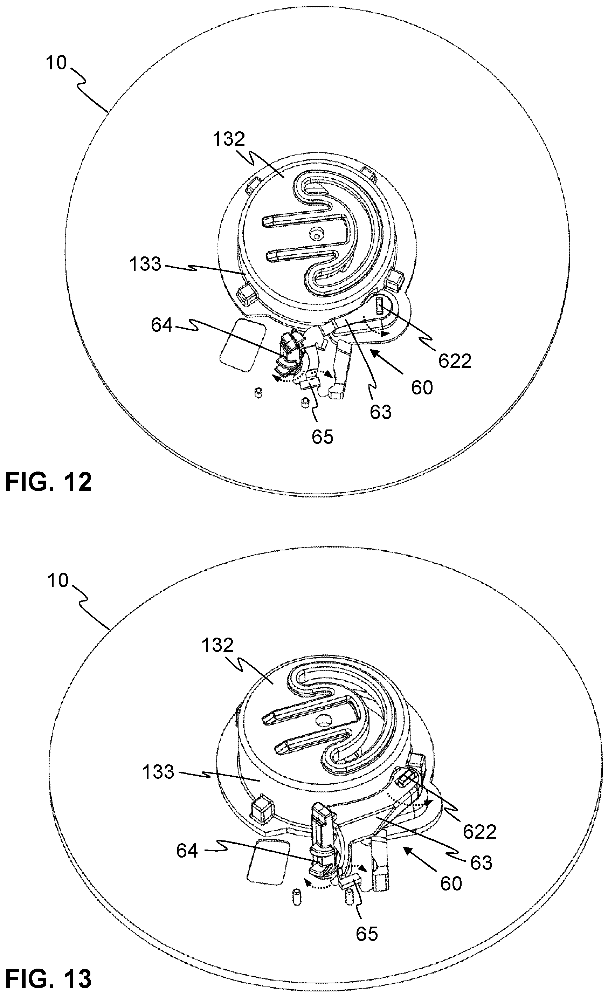

Fig. 9A and Fig. 9B illustrate a first embodiment of the locking mechanism 60 in a perspective view, wherein Fig. 9A shows the locked state and Fig. 9B shows the unlocked state. The latch 62 and the lever element 63 of this first embodiment are shown separately in an enlarged perspective view in Fig. 9C. Fig. 10A and Fig. 10B illustrate said first embodiment of the locking mechanism 60 in a top view, i.e. the viewing direction being the distal direction, wherein Fig. 10A shows the locked state, while Fig. 10B shows the unlocked state. Fig. 11A and Fig. 11B illustrate the first embodiment of the locking mechanism 60 in a bottom view, wherein Fig. 11A shows the locked state, while Fig. 11B shows the unlocked state. Fig. 12 and Fig. 13 show a second embodiment and a third embodiment of the locking mechanism 60, respectively, in a perspective view.

As visible in Fig. 11A the latch 62 comprises a tip 621 which engages with an indentation 38 located in the rim 36 of the rotating sleeve 30 (see also Fig. 3A and Fig. 7A). The latch 62 further comprises a shaft 622 extending through an opening in the base 10 parallel to the central axis A to reach the side of the base 10 opposite to the contact surface 11. To prevent any dirt from entering the patch-like medical device through said opening in the base, an O-ring 83 may arranged around the shaft to seal the opening (see Fig. 1 and Fig. 9C). On the side of the base 10 opposite to the contact surface 11, the shaft 622 of the latch 62 has an end, to which a lever element 63 is connected.

The lever element 63 may be shaped in a variety of ways; in particular, it may be an injection molded part (as shown in Fig. 9C and Fig. 13) or may be made of a sheet metal piece (as shown in Fig. 12). In all the embodiment shown here, the lever element 63 has an opening on a first end, in which the end of the shaft 622 is fixed. In the particularly advantageous first embodiment shown in Fig. 9C, said end has a star-like shaped profile which fits into the correspondingly shaped opening of the lever element 63 to enable a robust connection which is particularly suitable for enduring the torque exerted by the pre-loaded rotating sleeve 30 in the locked state, i.e. when the lever element 63 is clamped.

In the first embodiment of the locking mechanism 60 shown in Figs. 9A-11B, the actuation mechanism 82 comprises a motor-driven threaded shaft 821 and a transmission piece 822 that comprises a fork-like part (visible in Figs. 10A and 10B), wherein the threaded shaft 821 is in a gear-like engagement with a transmission piece 822. The actuation mechanism 82 further comprises a pivot arm 823 with a spherically shaped top end that is embraced by the fork-like part. Upon receiving a signal from the control circuitry, the threaded shaft 821 starts to rotate, thereby moving the transmission piece 822 from a first position to a second position. The fork-like part of the transmission piece 822 thereby drags along the spherically shaped top end of the pivot arm 823, causing the pivot arm 823 to pivot from a first pivot position to a second pivot position. The pivot arm 823 has a nose-like protrusion 824 (visible also in Figs. 2A-2C), which in the first pivot position pushes the clamp element 64 in distal direction (and thereby prevents it from moving) against a force exerted on the clamp element 64 by a resilient wire arrangement 825. As can be seen in Figs. 9A and 9B, the resilient wire arrangement 825 is partially wrapped around the sidewall 133 delimiting the recess 13 and urges the clamp element 64 to move in proximal direction from the first clamp position (where the clamp element 64 clamps the lever element 63) to the second clamp position (where the lever element 63 is released). When the pivot arm 823 is being moved to the second pivot position (Figs. 2B, 2C, 9B, 10B), the clamp element 64 moves in proximal direction, being linearly guided by a guiding structure 826 that is arranged on the base 10. As the clamp element 64 moves in proximal direction, it releases the lever element 63, which then pivots from the first lever element position to the second lever element position as the latch 62 is pushed radially outward by the rotating sleeve 30 that is urged to rotate by the pre-loaded spring 51.

In the embodiments of the lever element 63 shown in Fig. 12 and Fig. 13 the lever element 63 has a second end which is, in the first lever element position, biased in distal direction, in particular resiliently bent to abut against a stopper element 65, the stopper element 65 being attached to the base 10. In said first lever element position, which corresponds to the locked state of the locking mechanism, the lever element 63 prevents the latch 62 from disengaging from the rotating sleeve 30 by preventing rotation of the shaft 622, which is fixedly connected to the first end of the lever element 63. As indicated by the dotted arrows in Figs. 12 and 13, the lever element is movable from the first lever element position to a second lever element position in which the lever element allows the latch 62 to disengage from the rotating sleeve 30. The locking mechanism 60 further comprises a clamp element 64. The clamp element 64 is movable, here in particular rotatable, between a first clamp position in which the clamp element 64 clamps the lever element 63 in distal direction to the base 10 in the first lever element position, i.e. such that the lever element 63 abuts against the stopper element 65, and a second clamp position in which the clamp element 64 allows the lever element 63 to be released. The clamp element 64 comprises a nose, which, as the clamp element 64 is moved from the first clamp position to the second clamp position, bends the lever element 63 in proximal direction (in the embodiment shown in Fig. 12) or causes the lever element 63 to pivot (in the embodiment shown in Fig. 13) such that it does no longer abut against the stopper element 65. With the lever element 63 being free to move from the first lever element position to the second lever element position, the latch 62 is able to pivot as indicated by the dotted arrows in Fig. 11A and thus to disengage from the rim 36 of the rotating sleeve 30. The rotating sleeve 30 is then free to perform the helical movement. In cases where a spring 51 is driving said helical movement (as shown in the first and second embodiment of the patch-like medical device discussed above), the spring 51 is pre-loaded such that it exerts a force on the latch 62, in particular on the tip 621, when the needle protection sleeve 20 is in the first position and the locking mechanism 60 is in the locked state. This force urges the latch 62 to pivot radially outwards as indicated in Figs. 11A and 11B, but as long as the lever element 63 is clamped in the first lever element position in distal direction towards the base 10 by the clamp element 64, the latch 62 is prevented from pivoting by the clamped lever element 63. Once the lever element 63 is released, the latch 62 pivots outwards due to the force exerted by the spring and thereby also causes the lever element to move to the second position as indicated by the dashed arrows in Fig. 12 and Fig. 13.

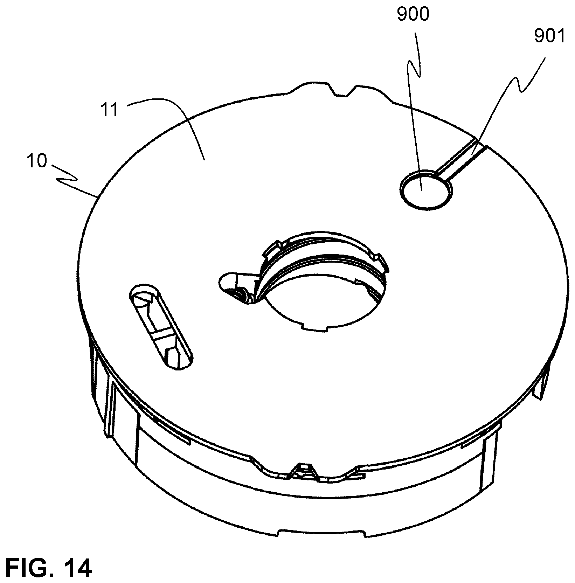

In the embodiment shown in Figs. 11A and 11B, the contact surface 11 has a recessed area 111 configured to receive the fixation ring 70 such that the fixation ring 70 is flush with the contact surface 11 when attached to the base 10 as shown in Fig. 1 and Fig. 6. As shown in Figs. 11A and 11B (where the fixation ring 70 is not shown) the latch is arranged in said recessed area 111. Once the fixation ring 70 is attached to the base 10, it at least partially covers the latch 62, such that the latch 62 does not enter into contact with the patient's skin. Alternatively, as shown in Fig. 14, the fixation ring may be formed by the base 10 itself instead of being a separate component.

Many variations of the components are conceivable without leaving the scope of the present invention and many features of the first and the second embodiment of the sleeves may be interchanged. In particular, it is possible to combine the different embodiments of the locking mechanism 60 and the trigger mechanism 80 including the actuation of the trigger mechanism 80 via one or more buttons 2 with each of the different embodiments of the sleeves shown either in Figs. 3A-3D or Figs. 7A-7D.

### Two-part configuration

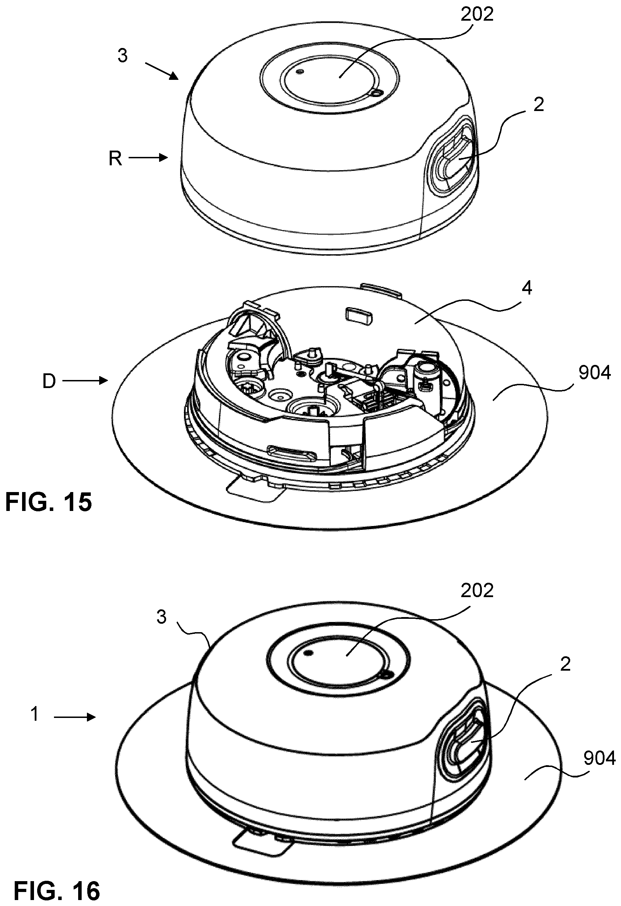

Preferably, the patch-like medical device 1 comprises a first device part D and a second device part R, wherein the first device part D and the second device part R are configured to be releasable coupled to each other. Fig. 15 shows a perspective view of the first device part D and of the second device part R being decoupled from each other, while Fig. 16 shows a perspective view of the first device part D and the second device part R being coupled to each other. As visible in Fig. 15, the first device part comprises the base 10, which acts as a housing lower part, and the second device part comprises the housing upper part 3, which is configured to be releasable coupled to the base 10. Preferably, the first device part D is disposable, i.e. configured for one-time use, and the second device part R is reusable, i.e. intended for multiple use.

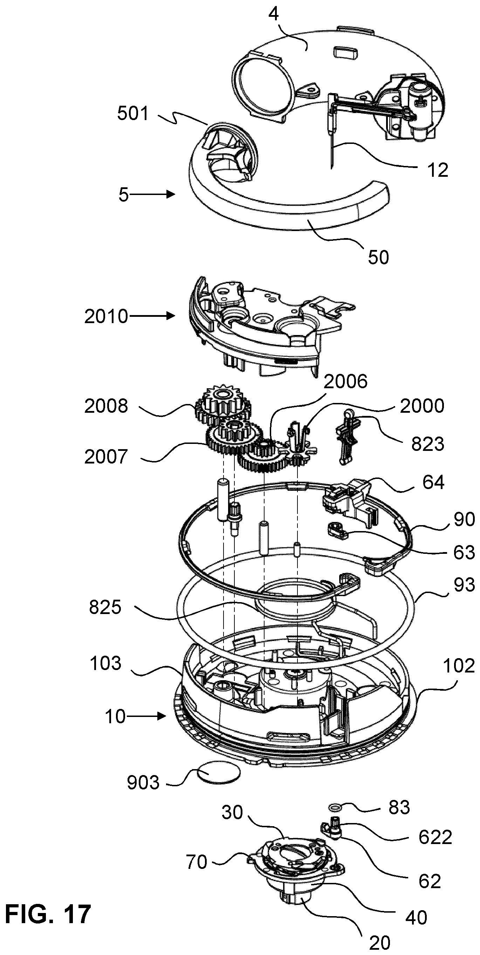

Fig. 17 shows an exploded view of the first device part D of the embodiment shown in Fig. 15. As visible in Fig. 17, the first device part D comprises a reservoir assembly with a reservoir 4 configured to contain a liquid substance to be administered to the patient, as well as a piston 5 to move the liquid substance within the reservoir 4, in particular to move the liquid substance towards and exit end of the reservoir 4, which is fluidically connected to the needle 12. To enable a compact design, the reservoir 4 has a toroidal shape and the piston 5 has a piston arm 50 that is curved accordingly in order to fit into the reservoir 4. Preferably, the piston 5 comprises a piston head 501 having a cross-section matching the cross-section of the reservoir 4 and comprises a seal element surrounding the piston head 501, the seal element gliding along an inner wall of the reservoir 4 when the piston head 501 is moved backward and forward, thereby sealing the reservoir 4 at one end.

### Reservoir assembly

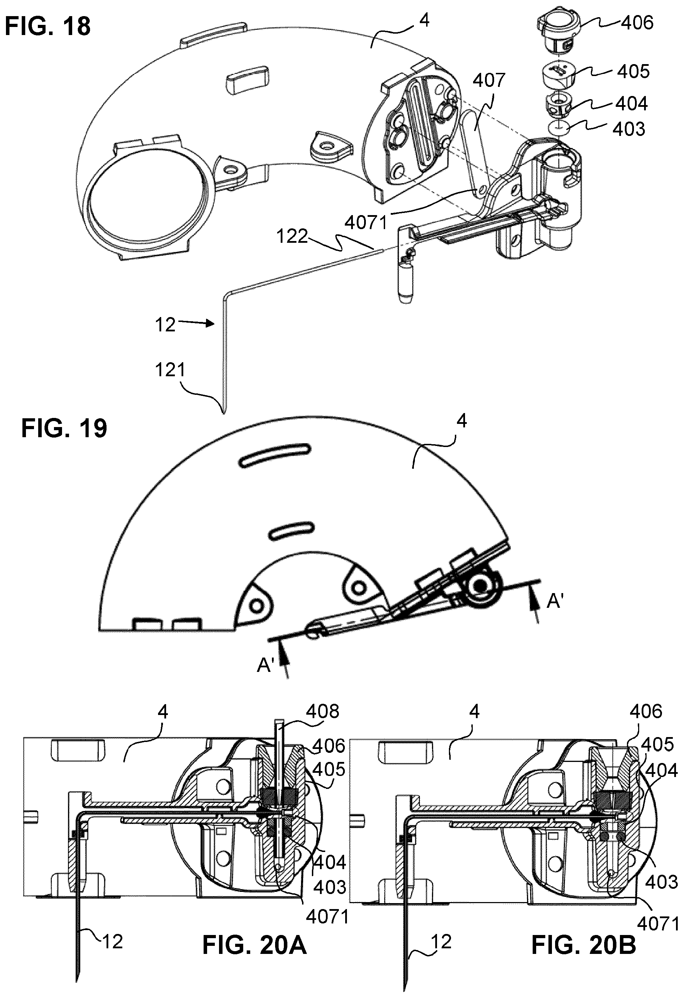

Fig. 18 shows an enlarged exploded view of the reservoir assembly and the needle 12. Fig. 19 shows a top view of the components shown in Fig. 18 and Figs. 20A and 20B show a sectional view along the sectional plane A'-A' marked in Fig. 19.

The reservoir assembly comprises a needle support structure 401, which is attached to the exit end of the reservoir 4, the needle support structure 401 holding the needle in a fixed position with respect to the reservoir 4. The needle 12 comprises a first needle portion extending along the central axis, the needle tip 121 that is configured for piercing the patient's skin being arranged at a distal end of the first portion, and a second needle portion extending perpendicularly to the first portion, wherein the second needle portion has a needle inlet end 122 which is arranged within the needle support structure 401. The reservoir assembly further comprises a filling gasket seal 407 being arranged between the exit end of the reservoir 4 and the needle support structure 401, the filling gasket seal 407 providing a passage hole 4071 through which fluids may enter or exit the reservoir 4, thereby providing a fluidic connection between the reservoir 4 and the needle support structure 401. The needle support structure comprises a filling casing 402 which provides a fluid path between the passage hole 4071 and the needle inlet end 121 and which is further configured to receive a filling needle 408 belonging to a separate filling aid device (not shown), via which filling needle 408 the liquid substance may be transferred from an external vial or cartridge into the reservoir 4.

In particular, as visible in Fig. 20A, the filling needle 408 may be inserted into the filling casing 402 from a top end of the filling casing 402. The passage hole 4071 is arranged adjacent to a bottom end of the filling casing 402, the bottom being arranged closer to the contact surface 11 of the base 10 of the patch-like medical device that the top end (see for instance Fig. 15). The reservoir assembly further comprises a filling O-ring 403, a filling insert 404, a septum 405 and a filling cover 406, which are all arranged within the filling casing 402. The filling cover 406 is arranged closest to the top end of the filling casing 402 and provides mechanical guiding when inserting the filling needle 408. The septum 405 is arranged below the filling cover 406 and has a septum slit, through which the filling needle 408 may be inserted, as shown in Fig. 20A. The septum 405 is arranged in the filling casing 402 in a compressed state so as to be self-sealing, i.e. such that the septum slit is substantially closed to prevent fluids from passing through the slit when no filling needle is inserted in the filling casing 402, as shown in Fig. 20B. In the embodiment shown here, the needle inlet end 122 of the needle 12 opens out into the filling casing 402 half way between the top end of the filling casing 402 and the bottom end of the filling casing 402. The needle inlet end 122 is arranged between the septum 405 and the filling O-ring 403, the filling O-ring 403 fitting tightly around the filling needle 408 when the latter is inserted as shown in Fig. 20A to prevent fluid communication between the reservoir 4 and the needle inlet 122 as long as the filling needle 408 is inside in the filling casing 402. The filling insert 404, which has a sleeve-like shape and is arranged between the filling O-ring 403 and the septum 405, keeps the filling O-ring 403 in place and prevents it from moving when the filling needle 408 is removed from the filling casing 402.

### Housing coupling mechanism

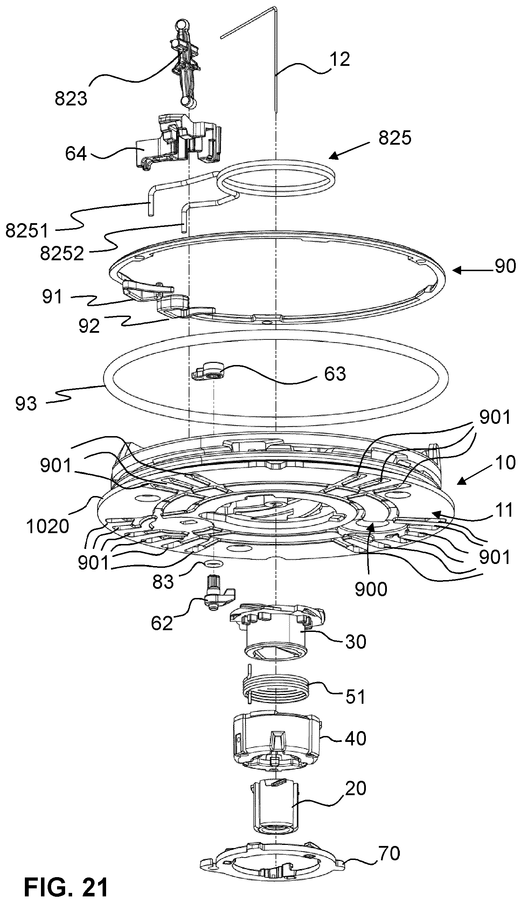

As shown in Fig. 17, the first device part D further comprises the base 10 and the needle protection mechanism including the locking mechanism 60. In Fig. 17, the sleeves of the needle protection mechanism are assembled. In Fig. 21, the needle protection sleeve 20, the rotating sleeve 30, the rotation lock sleeve 40 and the fixation ring 70 of the embodiments of Figs. 3A-3D are shown in an exploded view.

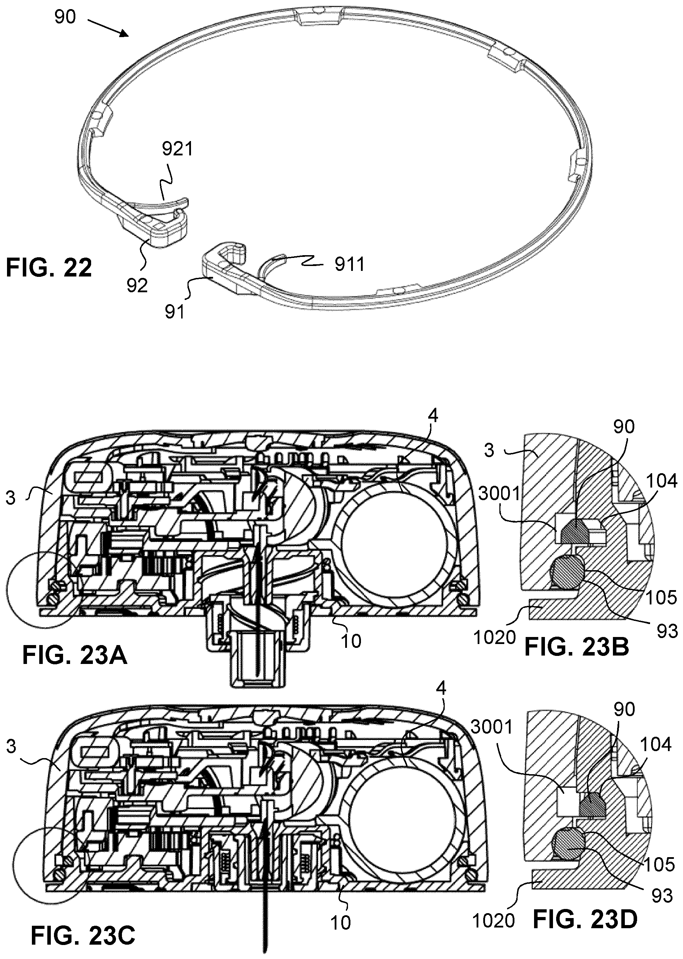

The patch-like medical device 1 further comprises a housing coupling mechanism for establishing a releasable coupling between the base 10 and the housing upper part 3. The housing coupling mechanism comprises a resilient retaining structure, which is arranged on the base 10. The resilient retaining structure comprises an open retaining ring 90, which is shown separately in Fig. 22. The open retaining ring 90 is an interrupted ring, i.e. the open retaining ring has a first end 91 and a second end 92, the first end 91 and the second end 92 being separated by a gap. The open retaining ring 90 has a resiliently variable diameter, i.e. the open retaining ring 90 is configured such that the gap between the first end 91 and the second end 92 may be varied.

In the embodiments shown here, the resilient wire arrangement 825, the clamp element 64 and the pivot arm 823 are part of the locking mechanism 60 (see description above), as well as of the housing coupling mechanism. The resilient wire arrangement 825 forms a first wire arm 8251 being connected to the first end 91 of the open retaining ring 90, and a second wire arm 8252 being connected to a second end 92 of the open retaining ring 90. The resilient wire arrangement 825 acts as a torsion spring and is biased to move the first and the second ends (91,92) of the open retaining ring 90 towards each other. The open retaining ring 90 itself has a first spring arm 911 and a second spring arm 912 that are configured to provide a counter-bias, i.e. to provide a spring force which pushes the first end 91 and the second end 92 of the open retaining ring away from each other. The first and second spring arm (911, 912) each have a fixed end attached to an inner surface of the open retaining ring, the inner surface facing radially inwards, and a free end pointing radially inwards and configured to abut against a stop structure (visible for instance in Fig. 15), wherein the stop structure preferably is the guiding structure 826, which is fixedly arranged on the base 10 and linearly guides the clamp element 64.

Figs. 23A-23D illustrate details of the mechanical engagement between the housing upper part 3 an the base 10. Fig. 23A and Fig. 23C show a sectional view of the first device part D and the second device part R in an assembled state, i.e. where the second device part R is placed on the first device part D. Fig. 23B shows an enlarged view of the encircled area marked in Fig. 23A and Fig. 23D shows an enlarged view of the encircled area marked in Fig. 23C. The resilient retaining structure is movable between a coupling position (shown in Figs. 23A and 23B) in which the housing upper part 3 is locked to the base 10, and a de-coupling position (shown in Figs. 23C and 23D) in which the housing upper part 3 and the base 10 can be separated from one another. The housing upper part 3 has a coupling recess 3001, the coupling recess 3001 being a radial groove formed in the housing upper part 3, in particular formed in a circumferentially extending wall of the housing upper part 3 and extending radially into the wall. As is visible in Figs. 23B and 23D, the groove is open towards an inside of the housing upper part 3. In the coupling position, the open retaining ring 90 radially reaches into the radial groove 3001 and thus locks the housing upper part 3 to the base 10. In the de-coupling position, the open retaining ring 90 does not reach into the radial groove 3001. The base 10 comprises a base plate 102, which is a substantially round disk providing the contact surface 11 to be directed towards the patient's skin on a first side. On a second side of the base plate 102 opposite to the first side, the base 10 comprises a base wall 103 extending perpendicularly to a surface normal of the base plate 102 and running circumferentially, thereby delimiting an inside of the base 10 in a radial direction. A base wall groove 104 is formed along at least a portion of the base wall 103 in an outer surface of the base wall facing radially outwards. The open retaining ring 90 is arranged partially within the base wall groove 104. The base wall 103 has two breakthroughs through which the open retaining ring 90 is arranged to pass such that the first and the second ends 91,92 of the open retaining ring 90, as well as the first and second spring arms 911,921 are located in the inside of the base 10. As visible in Figs. 23A-23D, the coupling recess 3001 and the base wall groove 104 are arranged so as to face each other when the housing upper part 3 and the base 10 are correctly assembled.

The base plate 102 has an outer rim 1020 extending radially beyond the outer surface of the base wall 103. As visible in Figs. 23B and 23D, the housing upper part 3 does not touch the outer rim 1020, i.e. a gap remains between the housing upper part 3 and the outer rim 1020 when the first device part D and the second device part R are in the assembled state. To nevertheless ensure watertight coupling between the base 10 an the housing upper part 3, the housing coupling mechanism further comprises a sealing ring 93 running circumferentially along the outer surface of the base wall 103, the sealing ring 93 being arranged on the base 10 in a base wall indentation 105 which is formed in the outer surface of the base wall 103, the base wall indentation 105 being located between the outer rim 1020 and the base wall groove 104. The housing upper part 3 has a ledge 3002 facing the inside of the housing upper part 3, the ledge 3002 being aligned with the base wall indentation 105 to accommodate the sealing ring 93 when the first device part D and the second device part R are in the assembled state.

In the present embodiment, the resilient retaining structure is in the coupling position prior to assembling the housing upper part 3 and the base 10, i.e. the open retaining ring 90 extends partially outside the base wall groove 104 as shown in Fig. 23B. To assemble the housing upper part 3 and the base 10, the housing upper part 3 is pushed onto the base, which initially causes the resilient open retaining ring 90 to be pushed radially inwards into the base wall groove 104. When the housing upper part 3 is pusher further onto the base 10, the open retaining ring 90 snaps radially outwards into the coupling recess 3001 due to the spring force provided via the first and second spring arms 911,921 of the open retaining ring 90.

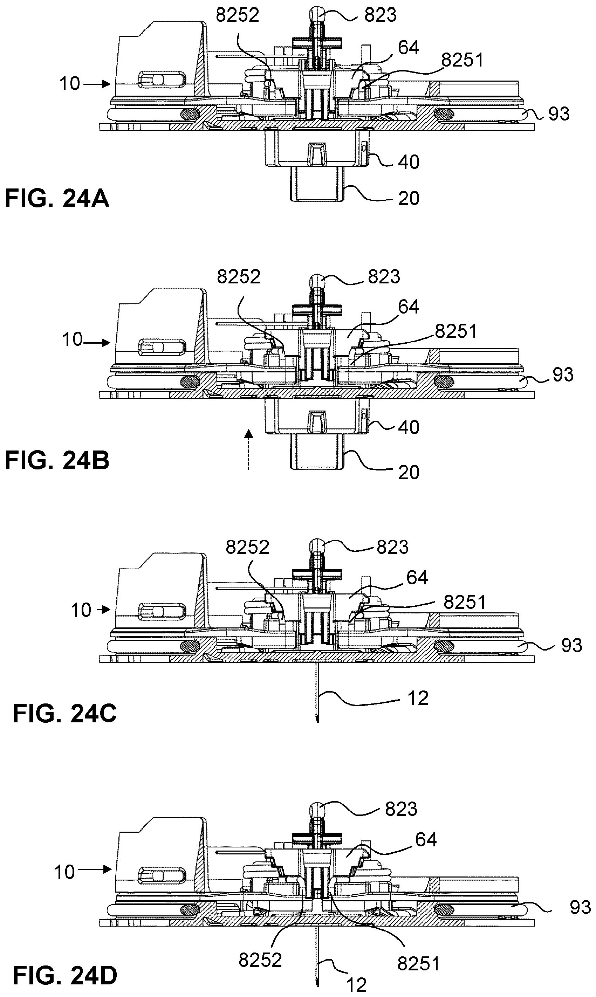

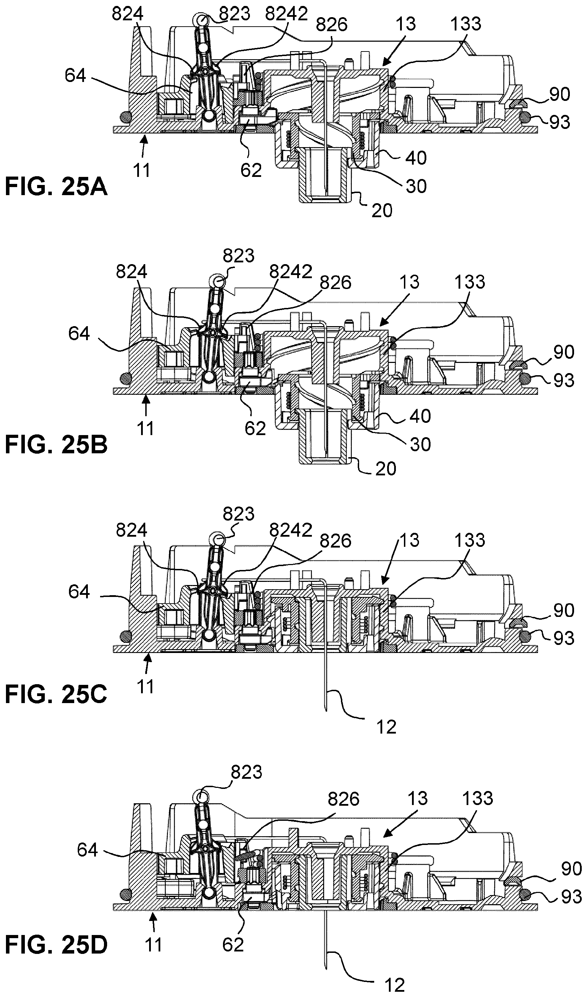

Figs. 24A-25D illustrate the relation between the housing coupling mechanism and a fourth embodiment of the locking mechanism 60 of the needle protection mechanism. Figs. 24A-24D show sectional views of the first device part D along a first sectional plane, while Figs. 25A-25D show sectional views of the first device part D along a second sectional plane being perpendicular to the first sectional plane. For the sake of better visibility, only components related to the housing coupling mechanism and the locking mechanism are shown in Figs. 24A-25D. In Figs. 24A and 25A, the fourth embodiment of the locking mechanism is in the locked state and the clamp element 64 is in the first clamp position, which corresponds to the clamp hold position in which the clamp element 64 prevents the first and second ends 91,92 of the open retaining ring 90 to move towards each other. As visible in Figs. 24A-24D, the clamp element 64 has a first step-like structure on a first side of the clamp element 64 which faces the first wire arm 8251 of the resilient wire arrangement 825, and a second step-like structure being symmetrical to the first step-like structure, but which faces the second wire arm 8252. When the clamp element 64 is in the first clamp position/clamp hold position as shown in Figs. 24A and 25A, the first and second wire arms 8251,8252 rest against a top step of the first and second step-like structure, respectively.

As visible in Fig. 25A, the spherically shaped top end of the pivot arm 823 in this fourth embodiment of the locking mechanism 60 points radially outwards when the pivot arm 823 is in the first pivot position, i.e. when the clamp element 64 is in the first clamp position (in contrast to the first embodiment of the locking mechanism, where the spherically shaped top end of the pivot arm 823 points radially inwards in the first pivot position as shown in Fig. 2A). Similar to the first embodiment, the pivot arm has a first nose-like protrusion 824 which pushes the clamp element 64 in distal direction against a force exerted on the clamp element 64 by the resilient wire arrangement 825.

Also similar to the first embodiment, when the pivot arm 823 is being moved to the second pivot position (Figs. 24B and 25B), the clamp element 64 moves in proximal direction to the second clamp position, being linearly guided by the guiding structure 826 that is arranged on the base 10. As the clamp element 64 moves in proximal direction, first and second wire arms 8251,8252 move to an intermediate step of the first and second step-like structure, respectively (see Fig. 24B). The first and second step-like structure on either side of the clamp element 64 are dimensioned such that the first and second wire arms 8251,8252 are at substantially the same distance from each other when resting against the top steps than when resting against the intermediate steps of the first and second step-like structure, respectively. Thus, when the clamp element is in the second position as shown in Figs. 24B and 25B, the open retaining ring 90, whose first and second ends 91,92 are attached to the first and second wire arms 8521,8252, respectively, keeps the same diameter as when the clamp element 64 is in the clamp hold position and hence stays in the coupling position. The second clamp position thus represents an intermediate position between the clamp hold position and the clamp release position, in which the resilient retaining structure is still in the coupling position.

Like in the first embodiment of the locking mechanism 60 however, as the clamp element 64 moves from the first clamp position to the second clamp position, it releases the lever element 63, which then pivots from the first lever element position to the second lever element position as the latch 62 is pushed radially outward by the rotating sleeve 30 that is urged to rotate by the pre-loaded spring 51. Like in the first embodiment shown in Figs. 2A-2C, the rotating sleeve 30 screws itself into the recess 13 while dragging along the rotation lock sleeve 40, which is hooked to the distal rim 39 of the rotating sleeve via three hooks 491. The dragged rotation lock sleeve 40 performs a linear movement in proximal direction, as it is prevented from rotating by the fixation ring 70, which in turn is attached to the base 10. The needle protection sleeve 20, which is prevented from rotating by the rotation lock sleeve 40, linearly move towards the base 10 while effectively getting screwed into the rotating sleeve 30, since the needle protection sleeve 20 is in a thread-like engagement with the rotating sleeve 30. Ultimately, the needle protection sleeve reaches its second position (shown in Figs. 24C and 25C), in which the needle tip 121 is exposed.

In the second clamp position/intermediate clamp position, the clamp element 64 is being pushed in distal direction by a second nose-like protrusion 8242 of the pivot arm 823 arranged radially opposite to the first nose-like protrusion 824 against the force exerted on the clamp element 64 by the resilient wire arrangement 825.

In Figs. 24D and 25D, the pivot arm 823 has been moved back to the first pivot position, and has thereby allowed the clamp element 64 to move further in proximal direction to reach the clamp release position, which has allowed the first and second wire arms 8251,8252 and thus the first and second ends 91,92 of the open retaining ring 90 to move towards each other and reach the de-coupling state depicted in Figs. 24D and 25D, where the open retaining ring 90 is contracted and does no longer protrude from the base wall 103.

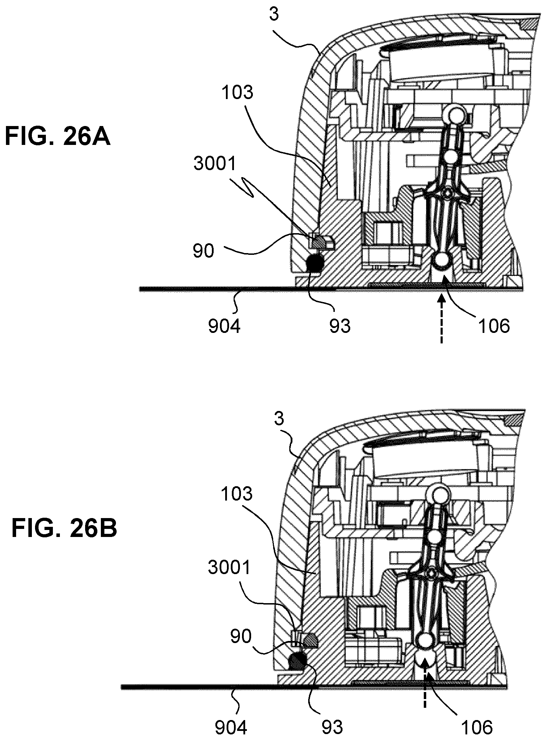

Figs. 26A and 26B show an enlarged portion of a sectional view of the patch-like medical device when the housing upper part 3 and the base 10 are in an assembled state to illustrate an emergency decoupling procedure. The emergency decoupling procedure may enable to decouple the housing upper part 3 from the base 10 in case the clamp element 64 is stuck in the second clamp position/intermediate clamp position. In such a case, a pointy object such as an unwrapped paper clip (not shown) may be inserted through an emergency decoupling hole 106 in the base plate 102 as indicated by the dashed arrows in Figs. 26A and 26B to push the pivot arm 823 in proximal direction, thereby releasing the clamp element 64 and allowing it to move to the clamp release position to allow the open retaining ring 90 to move from the coupling position (Fig. 26A) to the de-coupling position (Fig. 26B).

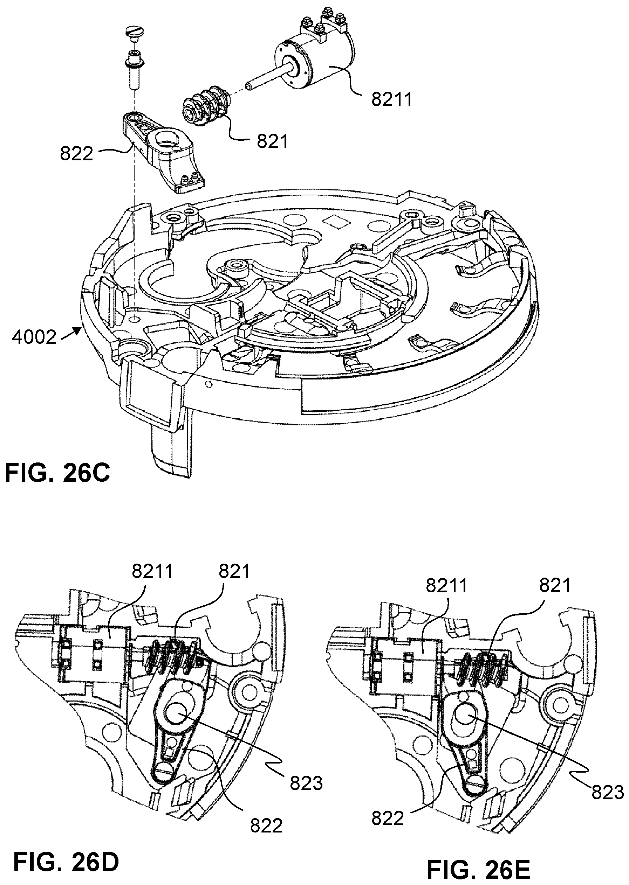

Figs. 26C-26E illustrates another embodiment of an actuation mechanism 82. Fig. 26D and Fig. 26E are top views of the actuation mechanism 82 shown in a perspective view in Fig. 26C. Similar to the embodiment of the actuation mechanism shown in Figs. 10A and 10B, the embodiment shown here also comprises the threaded shaft 821 and the transmission piece 822. The threaded shaft 821 is connected to a shaft motor 8211. The threaded shaft 821 with the shaft motor 8211 and the transmission piece 822 are arranged on a frame structure 4002, which is arranged in the second device part R. The transmission piece 822 shown in Figs. 26C-26E fulfils the same function as the one shown in Figs. 10A and 10B, i.e. it moves as the threaded shaft 821 rotates and thereby causes the pivot arm 823 to pivot from the first pivot position to the second pivot position, but is shaped slightly differently. Instead of having a fork-like part to embrace the spherically shaped top end of the pivot arm 823, the transmission piece 822 shown in Figs. 26C-26E has a hole configured to receive the spherically shaped top end of the pivot arm 823 when the first device part comprising the pivot arm 823 is connected to the second device part R. Fig. 26D shows the pivot arm 823 being in the first pivot position, i.e. corresponding to the position depicted in Figs. 25A and 25D. Fig. 26E shows the pivot arm 823 being in the second pivot position, i.e. corresponding to the position depicted in Figs. 25B and 25C.

### Drive mechanism and drive train

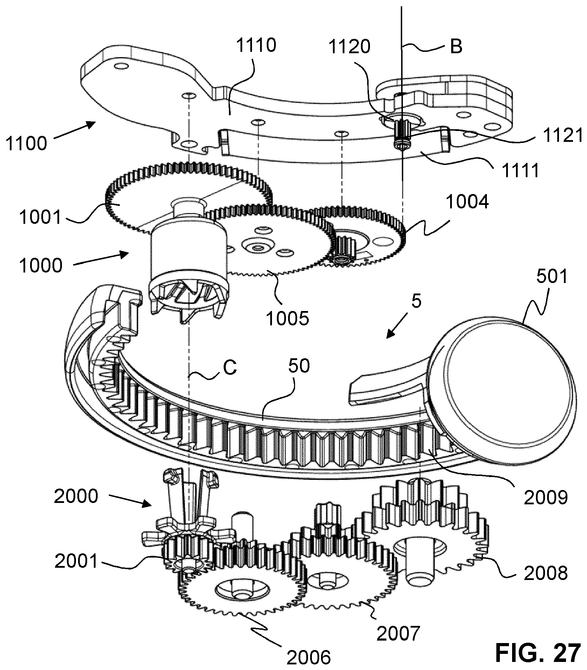

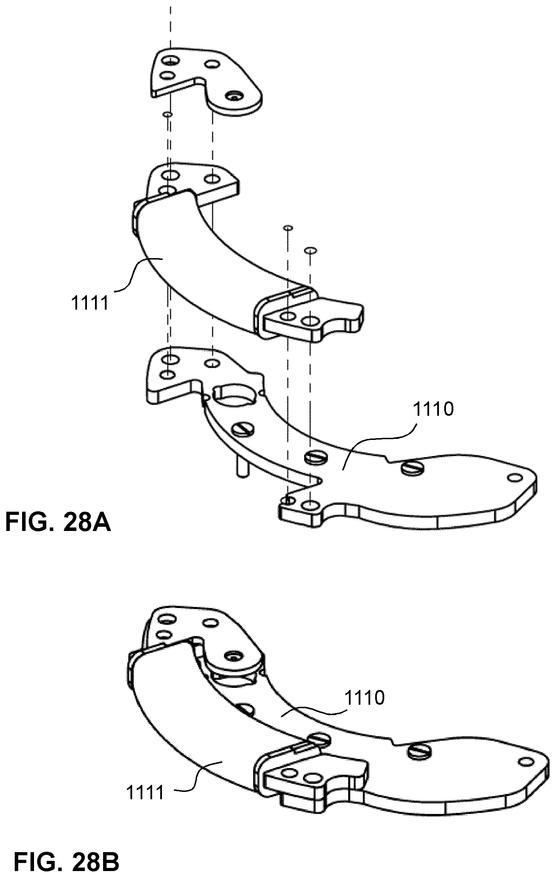

In order to drive the piston 5 within the reservoir 4, the embodiment of the patch-like medical device shown in Fig. 15 comprises a drive mechanism and a drive train, wherein the drive train transmits a mechanical movement from the drive mechanism to the piston 5. Fig. 27 shows an exploded view of the drive mechanism and the drive train of said embodiment of the patch-like medical device. The drive mechanism is a Lavet-type motor 1100, which comprises a stator part 1110, a rotor part 1120 and a coil 1111. The working principle of Lavet-type motors suitable for patch-like medical devices 1100 is described in EP2842586A1. The rotor part 1120 is made of magnetic material, in particular samarium-combalt Sm₂C0₁₇. A rotor gear wheel 1121 is concentrically attached to the rotor part 1120, so as to be rotatable in unison with the rotor part 1120 about a rotor axis B. Drive voltage pulses are applied to the coil 1111 to induce a step-wise rotation of the rotor part 1120. Fig. 28A shows an exploded view of the stator part 1100 and the coil 1111, while Fig. 28B shows the stator part 1100 and the coil 1111 in an assembled state. Both the stator part 110 and the coil 1111 have an arcuate shape to optimally fit within the substantially round housing upper part 3. The voltage pulses create a current in the coil 1111 and thus a magnetic field and a magnetic flux in the stator part 1120. If the polarity of the voltage pulse is such, that the created magnetic flux corresponds in its polarity to the flux created by the rotor, no torque is created at the rotor part 1120 and the rotor part 1120 stays in a rest position. If however the voltage pulse is of opposite polarity, the magnetic flux created by the coil also changes its sign and creates a torque in the rotor part 1120, turning it by 180°. Thus, the Lavet-type motor can be controlled by applying alternatively a positive and a negative voltage pulse to the coil. Each voltage pulse causes the rotor part 1120 to rotate by one step, which corresponds to a 180° turn of the rotor part 1120 about the rotor axis B.

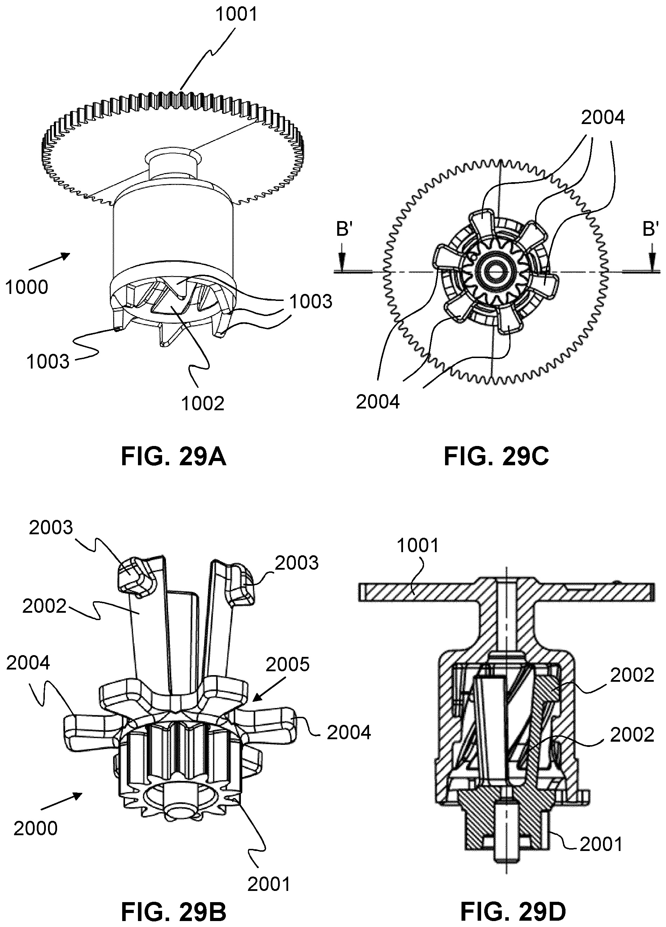

The embodiment of the drive train shown in Fig. 27 comprises a transmission gear system configured to be arranged in the second device part R, and a piston gear system configured to be arranged in the first device part D. The drive train further comprises a rotatable sleeve part 1000 (shown separately in Fig. 29A) and a rotatable plug part 2000 (shown separately in Fig. 29B). In the embodiment shown in Fig. 27, the rotatable sleeve part 1000 is configured to be arranged in the second device part R, while rotatable plug part 2000 is configured to be arranged in the first device part D. The sleeve part 1000 and the plug part 2000 are thus separate from each other when the first device part D and the second device part R are not coupled. The sleeve part 1000 and the plug part 2000 are engaged with each other in a final engagement state when the first device part D and the second device R part are coupled. Figs. 29C and 29D illustrate the final engagement state: Fig. 29C shows a top view, while Fig. 29D shows a sectional view along the sectional plane B'-B' marked in Fig. 29C. In the final engagement state, the sleeve part 1000 and the plug part 2000 are able to rotate in unison about a common rotation axis C to transmit the mechanical movement from the drive mechanism, in this embodiment the Lavet-type motor 1100, to the piston 5 to move the piston 5 within the reservoir 4. In particular, as visible in Fig. 27, the sleeve part 1000 comprises a sleeve gear wheel 1001 and the plug part 2000 comprises a plug gear wheel 2001. The transmission gear system comprises a first transmission gear wheel 1004 and a second transmission gear wheel 1005. The piston gear system comprises a first piston gear wheel 2006, a second piston gear wheel 2007 and a third piston gear wheel 2008. In the embodiment of the drive train shown in Fig. 27, the first and second transmission gear wheels 2004,2205 as well as the first, second, and third piston gear wheels 2006,2007,2008 are all configured as double-gear wheels.

The first transmission gear wheel 1004 is in meshing engagement with the rotor gear wheel 1121 to pick up the rotation movement of the rotor part 1120. The second transmission gear wheel 1005 is in meshing engagement with the first transmission gear wheel 1004 and simultaneously in meshing engagement with the sleeve gear wheel 1001 to transmit the movement from the first gear wheel 1004 to the sleeve part 1000. When the sleeve part 1000 and the plug part 2000 are in their final engagement state, the sleeve part 1000 entrains the plug part 2000 to rotate in unison. The plug gear wheel 2001 of the plug part 2000 is in meshing engagement with the first piston gear wheel 2006. The second piston gear wheel 2007 is in meshing engagement with both the first piston gear wheel 2006 and the third piston gear wheel 2008 to transmit the movement from the first piston gear wheel 2006 to the third piston gear wheel 2008. The third piston gear wheel 2008 is in meshing engagement with an internal gearing 2009 of the piston 5 to move the piston 5. The first, second, and third piston gear wheels 2006,2007,2008 of the piston gear system are arranged on the base plate 102 and are covered by a carrier structure 2010 (visible in Fig. 17), the carrier structure 2010 having a hole through which the plug part 2000 and the sleeve part 1000 can mate.

When the first device part and the second device part are brought into coupling in a direction parallel to the common rotation axis C, the sleeve part 1000 and the plug part 2000 are configured to forcibly rotate with respect to each other around the common rotation axis C until the final engagement state is reached.

In the embodiment of the sleeve part 1000 shown in Figs. 27 and 29A, the sleeve part 1000 has six alignment grooves extending helically about the common rotation axis C, the six alignment grooves being evenly distributed along a circumference of the inner surface of the sleeve part 1000.

In the embodiment of the plug part 2000 shown in Figs. 27 and 29B, the plug part has three alignment arms 2002, each having a lug 2003 configured to slide in one of the six alignment grooves 1002 of the sleeve part 1000 shown in Fig. 27 A when the first device part D and the second device part R are brought into coupling, the lugs 2003 being guided helically about the common rotation axis along the alignment grooves 1002 causing the plug part 2000 and the sleeve part 1000 to rotate with respect to each other until the final engagement state is reached. In the embodiment shown here, the lugs 2003 are located in every second alignment groove 1002 along the circumference of the inner surface of the sleeve part 1000 when the plug part 2000 and the sleeve part 1000 are in the final engagement state.

The lugs 2003 are each arranged at a free end of their respective alignment arm 2002, the free ends of the alignment arms 2002 entering into engagement with the sleeve part 1000 first when the first device part D and the second device part R are brought into coupling. The free ends of the alignment arms 2003 are resiliently bendable in a radial direction with respect to the common rotation axis C such that the lugs 2003 of the alignment arms 2002 and the alignment groove 1002 form a snap-fit connection when the plug part 2000 and the sleeve part 1000 are pushed into engagement with each other parallel to the common rotation axis C.

Each alignment arm 2002 has a fixed end that is attached to a star-shaped element, the star-shaped element having, as visible in Fig. 29C, six radial arms 2004 extending radially in a plane perpendicular to the common rotation axis C, thereby defining six tooth gaps 2005. As The sleeve part 1000 shown in Fig. 29A has tooth-like protrusions 1003 arranged at a first end of the sleeve part 1000, the first end of the sleeve part 1000 entering into engagement with the plug part 2000 first when the first device part and the second device part are brought into coupling. The six tooth-like protrusions 1003 extend in a direction parallel to the common rotation axis C and are each arranged in one of the tooth gaps 2005 between two of the radial arms 2004 of the star-shaped element when the plug part 2000 and the sleeve part 1000 are in the final engagement state, as visible in Fig. 29C.

### Venting arrangement

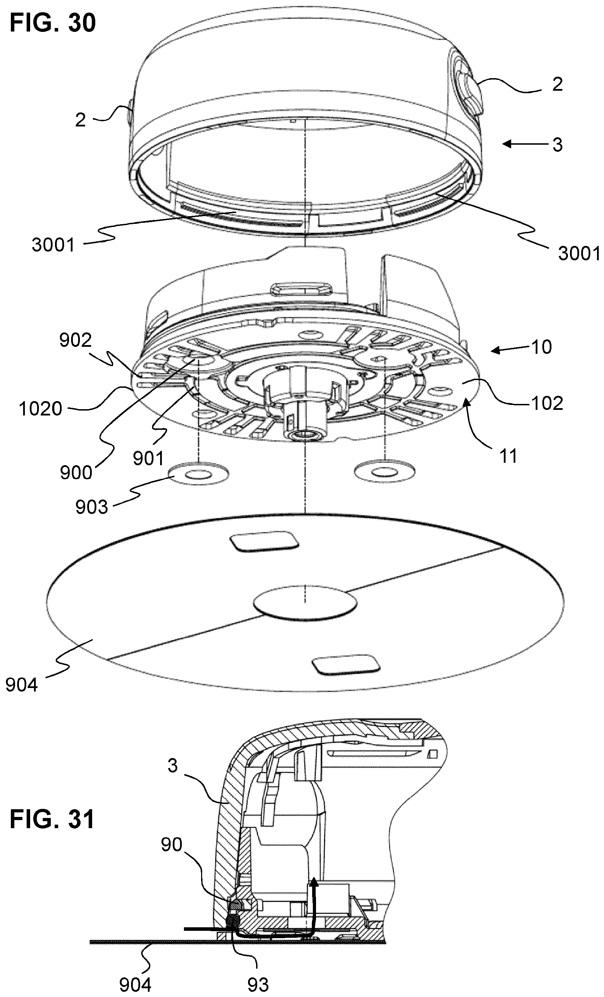

Fig. 30 shows an exploded view of the base part 10, the housing upper part 3 and a plaster 904, the base part 10 belonging to the first device part D of the embodiment of the patch-like medical device shown in Fig. 15, the housing upper part 3 and the plaster 904 belonging to the second device part R of the embodiment of the patch-like medical device shown in Fig. 15. The base 10 has a venting arrangement comprising a vent hole 900 extending through the base plate 102 to allow fluids to enter and/or exit an interior of the patch-like medical device, the interior of the patch-like medical device being defined by the first device part D and the second device part R when they are coupled together. The venting arrangement of the embodiment shown in Fig. 30 (also visible in Fig. 21) further comprises a plurality of vent channels 901 that are formed in the base plate, each vent channel 901 having a first end connected to the vent hole and a second end forming an inlet 902 located on an outer rim 1020 of the base plate 102. The vent hole 901 is arranged in a vent recess formed in the base plate 102 so that a gap remains between the vent hole 901 and the patient's skin when the patch-like medical device is attached to the patient's skin.

Preferably, as shown in Figs. 30 and 31, the vent hole 901 is covered by semi-permeable membrane 903, which allows gases, in particular air, to pass, but which blocks liquids, in particular water. The membrane 903 has a membrane rim, which is configured to be attached, in particular glued to the base plate 102. The membrane rim may be thicker than a center portion of the membrane 903.

The emergency decoupling hole 106 formed in the base plate 102 illustrated in Figs. 26A and 26B is also visible in Fig. 30. The emergency decoupling hole 106 may also be covered by such a semi-permeable membrane 903. Alternatively, the emergency decoupling hole 106 may be covered by a foil that is not permeable to fluids at all, but which may easily be pierced with a pointy object.

Furthermore, a plaster 904 is attached to the contact surface 11 of the base 10, the plaster 904 being formed by a sheet comprising a flexible carrier that is coated with a dermatologically compatible adhesive on a side of the carrier that is intended to be in contact with the patient's skin. The vent channels 901 are formed in the contact surface 11 as grooves being delimited on one side by the plaster 904. A gap remains between the membrane 903 covering the vent hole 901 and the plaster 904.

Fig. 31 shows a portion of a sectional view of the base 10 and the housing upper part 3 being in the coupled state. The arrow in Fig. 31 indicates a fluidic venting path which connects the interior of the patch-like medical device with the environment outside of the patch-like medical device. In the embodiment shown in Figs. 30 and 31, the inlets 902 are perforations arranged on an upper surface of the outer rim 1020 of the base plate 102, the upper surface of the base plate 102 being opposite to the contact surface 11, and air can enter/exit through the perforations, flow along the vent channels 901, enter/exit the gap between the plaster 904 and the membrane 903 and pass through the semi-permeable membrane 903 to reach/exit the interior of the patch-like medical device.

The vent channels 901 are preferably interconnected as shown in Fig. 30, which enables air to circulate from each of the inlets 902 towards the vent hole 900 (and back) to provide sufficient venting, but also to facilitate the evacuation of liquids, in particular water, which may enter through the inlets 902 if the patient wears the patch-like medical device while showering or swimming.

Figs. 1, 11A, 11B and 14 show a simpler venting arrangement having one vent channel 901 only leading to the vent hole 900 instead of the plurality of vent channels as shown in Fig. 30. Instead being a perforation, the inlet 902 is simply formed by the vent channel opening out on a side of the outer rim 1020 of the base plate 102.

Proper venting, as achieved by the venting arrangement described above, is important to prevent an under- or overpressure from occurring in the interior of the patch-like medical device, which could impact the functioning of the piston 5 within the reservoir 4, and which could thus impact the delivery of the liquid substance to the patient. Furthermore, the venting arrangement can also be necessary to ensure the functionality of electronic components, in particular batteries and acoustic elements, as will be described in the following section.

### Electronic components

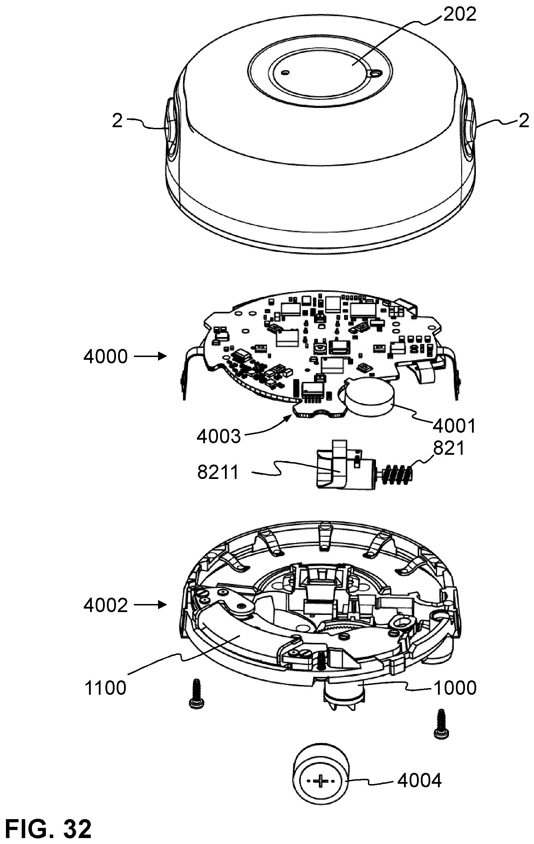

Fig. 32 shows a partially exploded view of components belonging to the second device part R, i.e. which are arranged within the housing upper part 3. In particular, Fig. 32 shows an electronic circuit board 4000, which comprises the control circuitry 81, which is configured to perform the functions schematically illustrated in Fig. 8, in particular to control the actuation mechanism 82. The motor-driven threaded shaft 821 of the actuation mechanism 82 (visible also in Figs. 10A, 10B and 26C-26E), which is responsible for causing the pivoting of the pivot arm, is also visible in Fig. 32.

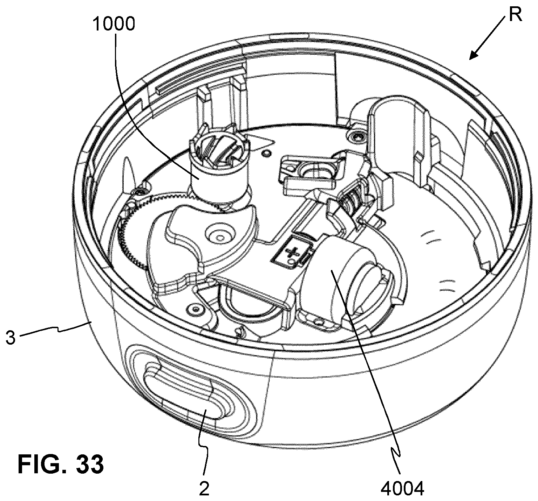

An acoustic element 4001 is attached to the electronic circuit board 4000, the acoustic element 4001 being for instance a magnetic transducer or a piezoelectrically driven buzzer, which is configured to emit audible alarm sounds to alert a user about a state of the patch-like medical device. Furthermore, Fig. 32 also shows the Lavet-type motor 1100, as well as the sleeve part 100 of the drive train. The Lavet-type motor 1100 is fixed to the frame structure 4002 (the frame structure 4002 without the Lavet-type motor is also visible in Fig. 26C). In the embodiment shown in Fig. 32, the electronic circuit board 4000 is substantially arranged between the frame structure 4002 and a top wall of the housing upper part 3. Electrical energy is provided by a non-replaceable battery 4003 and by a replaceable battery 4004. The non-replaceable battery 4003 is non-removable, but rechargeable. The replaceable battery 4004, which is preferably a zinc-air battery, is primarily providing electrical energy to the non-replaceable battery 4003, to charge the non-replaceable battery 4003 and to keep it above a desired charge threshold. The zinc-air battery needs oxygen to function properly. Air containing oxygen is preferably provided to the replaceable battery 4004 via the venting arrangement described above. The replaceable battery 4004 is accessible to the user when the second device part R is separated from the first device part D, and can be removed and exchanged. As can be understood by looking at Fig. 33, which shows a perspective view of the second device part R, the replaceable battery may be inserted into a slot provided by the frame structure 4002 and removed from said slot by grasping it with two fingers.

### Level sensor

In order to determine a filling state of the reservoir 4, the patch-like medical device further comprises a capacitive level sensor assembly 5000, which is arranged in the second device part R, and which is connected to the electronic circuit board 4000. When the first device part D and the second device part R are coupled, the capacitive level sensor assembly 5000 rests on the reservoir 4, in particular extending in a tangential plane with respect to the toroidally shaped reservoir, the tangential plane being parallel to the base plate 102.

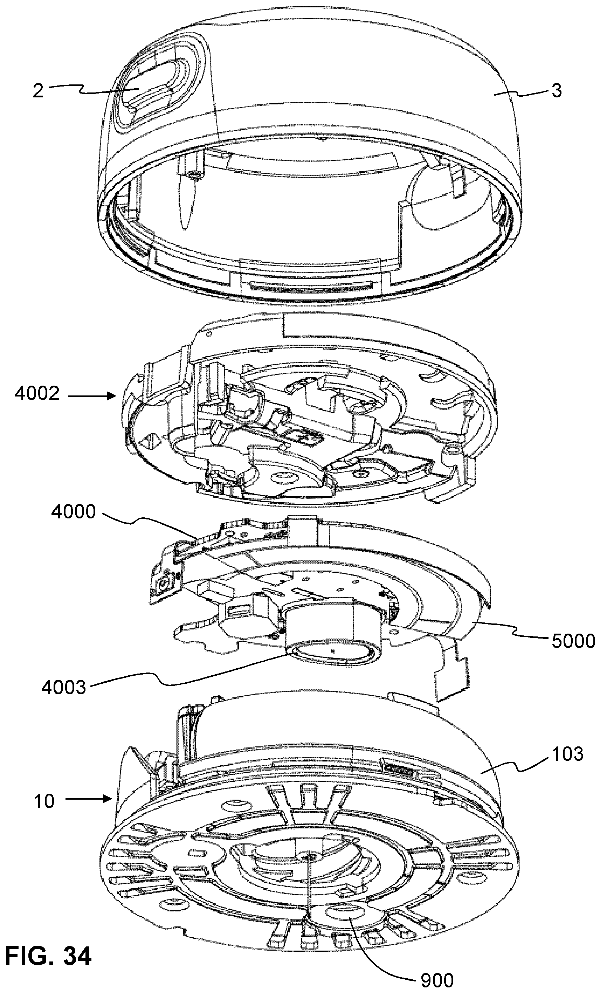

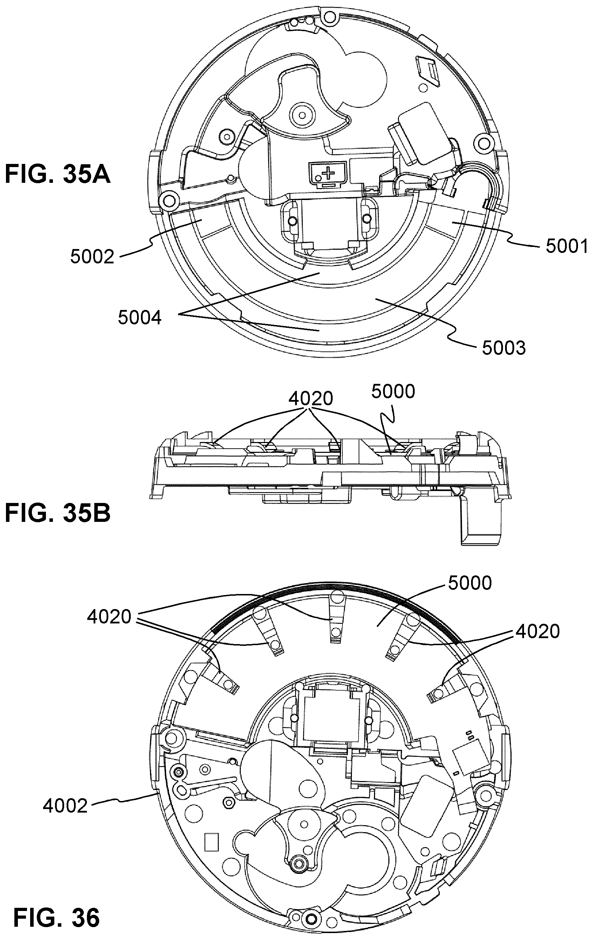

Fig. 34 shows an exploded view of the housing upper part 3, the frame structure 4002, the electronic circuit board 4000, the capacitive level sensor assembly 5000 and the base 10 with the reservoir 4. Fig. 35A shows a bottom view of the capacitive level sensor assembly 5000 being assembled with the frame structure 4002, the bottom view being defined here as a view of the side which is configured to face the reservoir 4, while Fig. 35B shows a side view of the capacitive level sensor assembly 5000 being assembled and with the frame structure 4002. Fig. 36 shows a top view of the capacitive level sensor assembly 500 being assembled with the frame structure 4002, the top view being defined here as a view of the side which is opposite to the side that faces the reservoir 4.

The frame structure 4002 comprises resilient clamp arms 4020, which extend from a rim of the frame structure radially inwards. In the example of the frame structure 4002 visible in Figs. 32, 34 and 36, there are five clamp arms 4020 being equally distributed along the rim over a length corresponding to a length of the reservoir 4. As visible in Figs. 35B and 36, the capacitive level sensor assembly 5000 is clamped within the frame structure 4002 and the resilient clamp arms 4020 are arranged to exert slight pressure on the capacitive level sensor assembly 5000 in distal direction to ensure that the capacitive level sensor assembly 5000 rests on the reservoir 4 free of mechanical play once the first device part D and the second device part R are coupled.

As marked in Figs. 34 and Fig. 35A, the capacitive level sensor assembly 5000 has three measurement regions: a liquid measurement region, which corresponds to a region of the reservoir 4 close to the exit end of the reservoir 4 and which is ideally always filled with the liquid substance to be administered to the patient (except towards the end of an administration process, when the piston 5 may enter the liquid measurement region to empty the reservoir 4), an environment measurement region, which corresponds to a region of the reservoir 4 located on a second end of the reservoir 4 opposite to the exit end and which should ideally always be free of the liquid substance to be administered to the patient, and a level measurement region, which is located between the liquid measurement region and the level measurement region. A liquid measurement electrode 5001 is arranged in the tangential plane and forms a co-planar plate capacitor together with the ground electrode 5004 to measure a liquid capacitance value in the liquid measurement region, an environment measurement electrode 5002 is arranged in the tangential plane and forms a co-planar plate capacitor together with the ground electrode 5004 to measure an environment capacitance value in the environment measurement region, and a level measurement electrode 5003 is arranged in the tangential plane between the liquid measurement electrode 5001 and the environment measurement electrode 5002 and forms a co-planar plate capacitor together with the ground electrode 5004 to measure a level capacitance value in the level measurement region measurement region. As shown in Fig. 35A, the ground electrode 5004 is shared by all three measurement regions and is split between an inner part and an outer part, the liquid measurement electrode 5001, the level measurement electrode 5003 and the environment measurement electrode 5002 being curved according to a curvature of the toroidal reservoir 4 and being arranged radially between the inner part and the outer part of the ground electrode 5004.

In an idealized theoretical model, the following conditions would be met:
- the liquid measurement region is always completely full,
- the environment measurement region is always completely empty,
- the environment measurement region and the liquid measurement region have identical geometrical dimensions,
- the environment measurement region, the level measurement region and the liquid measurement region are adjacent to each other, in particular without any gap between the level measurement region and the liquid measurement region, and the level measurement region together with the liquid measurement region perfectly cover the maximum fillable volume of the reservoir 4.

In practice, the liquid substance may at least partially enter into the environment measurement region 5002 when the reservoir 4 is full, i.e. a presence of the liquid substance may be sensed in the environment measurement region 5002 in the beginning of the liquid administration process when starting with a full reservoir 4. In addition, the liquid measurement region 5001 may be at least partially depleted when the piston 5 reaches the exit end of the reservoir 4, i.e. the capacitance value measured in the liquid measurement region may change towards the end of the liquid administration process when the reservoir 4 is close to being empty. Taking into account these two effects, a filling level *h* (in percentage) of the liquid substance within the reservoir 4 may then be determined using the following formula: $h = \frac{{C}_{level} + {C}_{liquid} + {C}_{env} - {C}_{env} \left({E}_{factor}+{EL}_{factor}\right) - {C}_{env _ empty}}{{C}_{liquid} \left({L}_{factor}+1\right) + {C}_{env _ full} - {C}_{env} \left({E}_{factor}+{EL}_{factor}\right) - {C}_{env _ empty}} ∗ 100$ wherein:
*Cₗₑᵥₑₗ* : capacitance measured in the level measurement region
*C_{liquid}* : capacitance measured in the liquid measurement region
*Cₑₙᵥ* : capacitance measured in the environment measurement region
*C_{env_empty}* : capacitance in the environment measurement region without any liquid in the environment measurement region (reference value)
*C_{env_full}:* capacitance in the environment measurement region when the reservoir is completely full (reference value)
*C_{liquid_empty}* : capacitance in the liquid measurement region without any liquid in the liquid measurement region, i.e. when the reservoir is completely empty (reference value)
*C_{liquid_full}*: capacitance in the liquid measurement region when the liquid measurement region is completely filled with liquid (reference value)
*C_{level_empty}* : capacitance measured in the level measurement region without any liquid in the level measurement region (reference value)
*C_{level_full}*: capacitance measured in the level measurement region when the level measurement region is completely filled with liquid (reference value),
and wherein the following proportionality factors (constants) are used based on the parameters defined above: ${L}_{factor} = \frac{{C}_{level _ full}}{{C}_{liquid _ full}}$ ${E}_{factor} = \frac{{C}_{level _ empty}}{{C}_{env _ empty}}$ ${EL}_{factor} = \frac{{C}_{liquid _ empty}}{{C}_{env _ empty}}$

In practice, to allow for a better accuracy of the filling level estimation when the piston 5 reaches the exit end of the reservoir 4, an edge detection may be used to detect a point where capacitance measured in the liquid measurement region 5001 starts to decrease in relation to the total summed capacitance of all three measurement regions. After the edge has been detected, a remaining filling level may be counted down using the motor steps executed. A blending function may be used in order to obtain a smooth transition between the filling level estimation obtained using the capacitance measurement in the three measurement regions, and the filling level obtained using the edge detection in combination with the motor steps.

### Occlusion detection

Ensuring proper delivery of the liquid substance to the patient is crucial to prevent unintentional under- or overdosing. Thus, it is important to be able to detect occlusion events, i.e. obstructions in the fluidic path that may occur between the reservoir 4 and the needle tip 121 or at the needle tip 121.

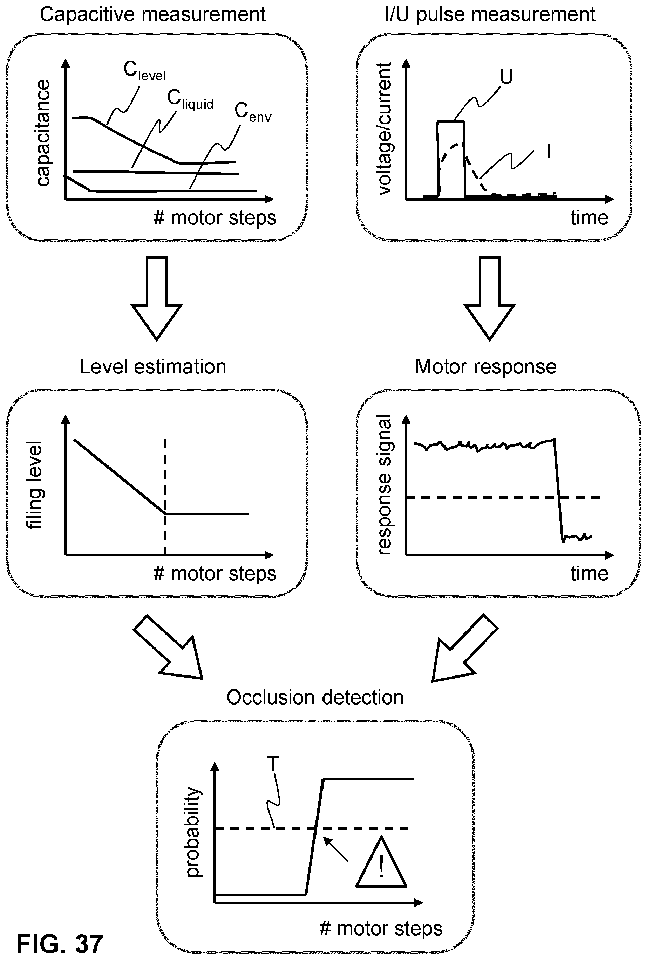

Fig. 37 schematically illustrates an example of a method for determining an occlusion probability, which combines measurements carried out on the stepping motor with measurements carried out using the capacitive level sensor assembly.

In particular, four input probabilities resulting from four different methods are computed and a combined probability is computed by OR-ring (i.e. using a logic "OR") such that the output is the probability that that none of the four input probabilities would declare occlusion.

In a final step, the combined probability is then compared to a probability threshold T (see Fig. 37) to determine whether an occlusion warning signal is to be emitted or not.

The four methods are a level-estimation method, a lost-step detection method, a motor-torque method, and a rotor vibration detection method.

The level-estimation method comprises:
a. measuring an absolute change Δ*h*_{abs} in filling level *h* based on the capacitance Cₗₑᵥₑₗ in the level measurement region 5003, the capacitance Cₑₙᵥ measured in the environment region 5002, and the capacitance C_{liquid} measured in the liquid measurement region 5001 during a pre-determined number of steps of the stepping motor, preferably using the formula for the level *h* described above;
b. computing a change Δ*h*ₛₜₑₚ of the filling level per step of the stepping motor based on the absolute change Δ*h*_{abs} and the pre-determined number of steps;
c. computing a level-method-based probability of occlusion *P*_{L} based on a comparison of the computed average change Δ*h*ₛₜₑₚ per step of the stepping motor with a first reference change value Δ*h*_{ref}=-1cIU corresponding to a non-occluded state, and with a second reference change value Δ*h*_{occ}= 0 clU corresponding to an occluded state, wherein the unit "1 clU" corresponds to a volume of the liquid substance delivered by one motor step in a non-occluded state of the patch-like medical device.

Lost motor steps may be a strong indication of occlusion or other malfunctions and a method based on detecting lost motor steps may thus be highly reliable.

The lost-step-detection method comprises:
a. measuring a coil flux of the Lavet-type motor;
b. comparing the measured coil flux with a flux threshold value, the flux threshold value being dependent on motor parameters;
c. determining a lost-step-based probability of occlusion *P*_{S} based on a comparison of the measured coil flux with the flux threshold value, in particular setting the probability of occlusion *P*_{S} to 1 if the measured coil flux is below the flux threshold value for at least a pre-determined number N of motor steps among a total pre-determined number M motor steps, where N e.g. could be 4 and M could be 100.

Due to an elasticity of the pump mechanism, the detection of occlusion events using the lost-step-detection method may be delayed compared to other methods.

An estimation of a torque value of the motor based on internal energy may also be used.

The motor-torque method comprises:
a. obtaining a motor response signal over a pre-determined period of time;
b. determining internal energy data based on the motor response signal as a function of time;
c. applying a noise filter to the internal energy data;
d. detecting a rise in the filtered internal energy data;
e. computing a motor-torque-based probability of occlusion *P*_{T}, the motor-torque-based probability of occlusion *P*_{T} being proportional to a steepness of the rise in the filtered internal energy data.

Furthermore, a rotor vibration detection method may be used, which is based on evaluating internal energy data of the motor response signal during a passive phase of the motor, in particular evaluating vibrations, i.e. back-and forth movements of the rotor part at the end of a motor step. These vibrations are visible as a change in internal energy. In case the motor is running at its limits during the motor step, the back-and forth movements of the rotor part at the end of the motor step are typically strongly damped or inexistent, which may point towards an occlusion event.

The rotor vibration detection method comprises:
a. obtaining a motor response signal during a pre-determined time interval after an actively driven part of a motor step has been completed;
b. determining internal energy data based on the motor response signal during the pre-determined time interval after the motor step has been completed;
c. detecting a change in the internal energy data in the pre-determined time interval after an actively driven part of a motor step has been completed;
d. computing a vibration-based probability of occlusion *P*_{V}, the vibration-based probability of occlusion *P*_{V} being based on the detected change in the internal energy data.

The coil flux *ψ*(*t*) mentioned above depends on an integration time t and is defined as: $ψ \left(t\right) = {\int }_{0}^{t} \left(U\left(t′\right)-RI\left(t′\right)\right) dt ′$

The internal energy W(t) mentioned above is dependent on an integration time t and is defined as $W \left(t\right) = {\int }_{0}^{t} \left(U\left(t′\right)I\left(t′\right)-{RI}^{2}\left(t′\right)\right) dt ′$ wherein U(t') is the voltage across the motor at time t', l(t') is the motor current at time t' and R is the ohmic resistance of the coil windings of the motor, which is a constant value. A motor step preferably has a total duration of 50 milliseconds, wherein the actively driven part of a motor step preferably has a duration of 7.5 milliseconds.

It is of course conceivable to combine only a selection of the four methods disclosed here to determine the combined probability value.

### User interaction

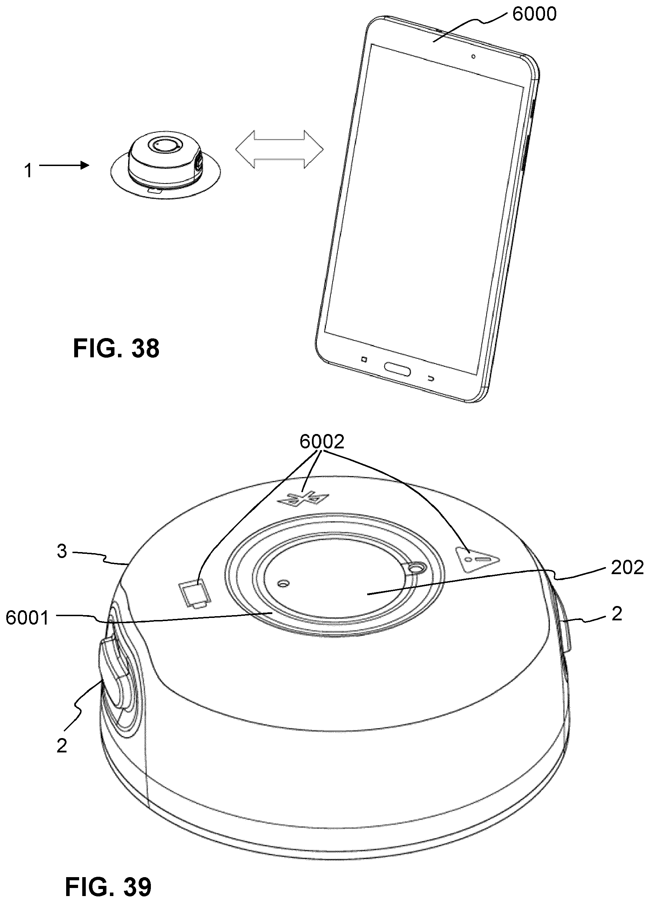

The patch-like medical device 1 is preferably wirelessly connectable to a remote control device 6000, in particular a tablet computer and/or smartphone, to program and control the device, as symbolically illustrated in Fig. 38. For this purpose, the control circuitry 81 comprises a communication module, preferably configured to communicate to the remote control device 6000 via Bluetooth.

Fig. 39 shows an embodiment of the housing upper part 3, which has no display, but which comprises alert symbols 6002 that are configured to light up to inform the users of a pairing status (e.g. using a Bluetooth symbol), of a status of the batteries (e.g. using a battery symbol), or of a general malfunction (e.g. using a warning symbol). Additionally, the housing upper part 3 comprise a light ring 6001, which is preferably configured to emit light in different colors and with different blinking patterns depending on a status of the patch-like medical device. The light for the alert symbols 6002 and the light ring 6001 is preferably provided by light-emitting diodes (LEDs)

In addition to visual feedback, the patch-like medical device may also be configured to emit sounds, in particular a series of tones at a variety of different acoustic frequencies, and/or vibrations using the acoustic element 4001 and or a separate vibrational element.

The embodiment of the housing upper part furthermore shows two buttons 2 arranged opposite to each other on either side of the housing upper part 3, as well as a third button (top button 202), which is arranged on top of the housing upper part 3 within the light ring 6001.

**LIST OF REFERENCE SIGNS**

| | | | |
|---|---|---|---|
| 1 | patch-like medical device | 131 | internal thread of the recess |
| 2 | button | 132 | top wall of the recess |
| 202 | top button | 133 | side wall of the recess |
| 3 | housing upper part | 14 | cylindrical element |
| 3001 | coupling recess of the housing upper part | 141 | helical grooves |
| | | 20 | needle protection sleeve |
| 3002 | ledge | 21 | external thread portion (of the needle protection sleeve) |
| 4 | reservoir | | |
| 401 | needle support structure | 22 | internal thread (of the needle protection sleeve) |
| 402 | filling casing | | |
| 403 | filling O-ring | 23 | longitudinal groove |
| 404 | filling insert | 24 | guide cam |
| 405 | septum | 25 | protection sleeve contact surface |
| 406 | filling cover | | |
| 407 | filling gasket | 26 | indentations |
| 4071 | passage hole | 251 | needle hole |
| 408 | filling needle | 252 | opening |
| 5 | piston | 30 | rotating sleeve |
| 50 | piston arm | 301 | protrusion of the rotating sleeve |
| 501 | piston head | | |
| 10 | base | 31 | second bore |
| 101 | protrusion of the base | 32 | thread portion |
| 102 | base plate | 33 | thread portion (of the rotating sleeve) |
| 1020 | outer rim of the base plate | | |
| 103 | base wall | 34 | internal thread |
| 104 | base wall groove | 35 | external thread portions |
| 105 | base wall indentation | 36 | proximal rim of the rotating sleeve |
| 106 | emergency decoupling hole | | |
| 11 | contact surface | 37 | rotating sleeve front wall |
| 111 | recessed area | 38 | indentation |
| 12 | needle | 39 | distal rim of the rotating sleeve |
| 121 | needle tip | | |
| 122 | needle inlet end | 371 | central opening |
| 13 | recess | 372 | bore |
| 373 | arched opening | 823 | pivot arm |
| 40 | rotation lock sleeve | 824 | first nose-like protrusion |
| 41 | first bore | 8242 | second nose-like protrusion |
| 42 | guide cam | 825 | resilient wire arrangement |
| 43 | longitudinal groove | 8251 | first wire arm |
| 431 | end facet of the longitudinal groove | 8252 | second wire arm |
| | | 826 | guiding structure |
| 44 | longitudinal ridge | 83 | O-ring |
| 45 | rim portions of the rotation lock sleeve | 90 | open retaining ring |
| | | 91 | first end |
| 46 | rotation lock sleeve front wall | 911 | first spring arm |
| 47 | opening | 92 | second end |
| 471 | edge of the opening | 921 | second spring arm |
| 48 | notch | 93 | sealing ring |
| 49 | inner wall of the rotation lock sleeve | 900 | vent hole |
| | | 901 | vent channel |
| 491 | hook | 902 | inlet |
| 51 | spring | 903 | membrane |
| 511 | first end of the spring | 904 | plaster |
| 512 | second end of the spring | 1000 | sleeve part |
| 60 | locking mechanism | 1001 | sleeve gear wheel |
| 62 | latch | 1002 | alignment groove |
| 621 | tip | 1003 | tooth-like protrusion |
| 622 | shaft | 1004 | first transmission gear wheel |
| 63 | lever element | 1005 | second transmission gear wheel |
| 64 | clamp element | | |
| 65 | stopper element | 1100 | Lavet-type motor |
| 70 | fixation ring | 1110 | stator part |
| 701 | inner edge of the fixation ring | 1111 | coil |
| 71 | notch | 1120 | rotor part |
| 80 | trigger mechanism | 1121 | rotor gear wheel |
| 81 | control circuitry | 2000 | plug part |
| 82 | actuation mechanism | 2001 | plug gear wheel |
| 821 | threaded shaft | 2002 | alignment arm |
| 8211 | shaft motor | 2003 | lug |
| 822 | transmission piece | 2004 | radial arms |
| 2005 | tooth gap | 5001 | liquid measurement region |
| 2006 | first piston gear wheel | 5002 | environment measurement region |
| 2007 | second piston gear wheel | | |
| 2008 | third piston gear wheel | 5003 | level measurement region |
| 2009 | internal gearing | 5004 | ground electrode |
| 2010 | carrier structure | 6000 | remote control device |
| 4000 | electronic circuit board | 6001 | light ring |
| 4001 | acoustic element | 6002 | alert symbols |
| 4002 | frame structure | A | central axis |
| 4020 | clamp arms | B | rotor axis |
| 4003 | non-replaceable battery | C | common rotation axis |
| 4004 | replaceable battery | D | first device part |
| 5000 | capacitive level sensor assembly | R | second device part |

## Claims

1. A patch-like medical device comprising:
a base (10) having a contact surface (11) configured to be directed towards a patient's skin;
a needle (12) fixedly positioned relative to the base (10), the needle (12) having a needle tip (121) which protrudes from the contact surface (11) and which is configured for piercing the patient's skin;
a needle protection sleeve (20) movable along a central axis (A) from a first position to a second position, the needle protection sleeve (20) extending beyond the needle tip (121) to prevent the needle tip (121) from piercing the patient's skin in the first position, and exposing the needle tip (121) to enable piercing the patient's skin in the second position;
**characterised in that**, the patch-like medical device further comprises :
a rotating sleeve (30) movable along the central axis (A), the rotating sleeve (30) being in a thread-like engagement with the needle protection sleeve (20) such that a helical movement of the rotating sleeve (30) with respect to the base (10) causes the needle protection sleeve to move along the central axis (A) from the first position to the second position;
a rotation lock sleeve (40) movable along the central axis (A) while being rotationally fixed with respect to the base, the rotation lock sleeve (40) being in engagement with the needle protection sleeve (20) such that the needle protection sleeve (20) is prevented from rotating with respect to the base (10) when the needle protection sleeve (20) moves from the first position to the second position.

2. The patch-like medical device of claim 1,
wherein the rotating sleeve (30) at least partially protrudes from the contact surface (11) when the needle protection sleeve (20) is in the first position,
wherein the rotating sleeve (30) axially retracts towards the contact surface (11) along the central axis (A) during its helical movement, and wherein the needle protection sleeve (20) is configured to axially move relative to the rotating sleeve (30) along the central axis (A) during the helical movement of the rotating sleeve (30).

3. The patch-like medical device of claim 1 or 2,
wherein in the second position, the needle protection sleeve (20), the rotating sleeve (30) and the rotation lock sleeve (40) are each at least partially, preferably fully, retracted inside the patch-like medical device on a side that faces away from the contact surface (11).

4. The patch-like medical device of any one of the preceding claims,
wherein the needle protection sleeve (20) is at least partially received radially inside the rotating sleeve (30),
wherein the base (10) defines a recess (13) on a side that faces away from the contact surface, the recess being delimited by a side wall (133) in a radial direction, and
wherein the rotating sleeve (30) and the side wall (133) are in a thread-like engagement with each other.

5. The patch-like medical device of any one of claims 1-3,
wherein the rotating sleeve (30) is at least partially received radially inside the needle protection sleeve (20),
wherein the base (10) comprises a central guide element (14) extending along the central axis (A),
wherein the central guide element (14) is at least partially received radially inside the rotating sleeve (30),
and wherein the rotating sleeve (30) and the central guide element (14) are in a thread-like engagement with each other.

6. The patch-like medical device of any one of the preceding claims,
wherein one of the needle protection sleeve (20) and the rotation lock sleeve (40) comprises a longitudinal groove (23,43) extending parallel to the central axis (A) and wherein the other one of the needle protection sleeve (20) and the rotation lock sleeve (40) comprises a guide cam (24,42) configured to slide in said longitudinal groove (23,43) such that the protection sleeve (20) is prevented from rotating with respect to the base (10) when moving from the first position to the second position.

7. The patch-like medical device of any one of the preceding claims, further comprising a fixation element that is fixedly attached to the base (10) and is in engagement with the rotation lock sleeve (40) such that the rotation lock sleeve is prevented from rotating relative to the base, wherein the fixation element is preferably configured as a fixation ring (70).

8. The patch-like medical device of any one of the preceding claims, further comprising a spring (51) configured to drive the helical movement of the rotating sleeve (30), the spring (51) being pre-loaded when the needle protection sleeve (20) is in the first position, wherein the spring (51) at least partially relaxes when the needle protection sleeve (20) moves from the first position to the second position.

9. The patch-like medical device of any one of the preceding claims, further comprising a locking mechanism (60) having a locked state in which the locking mechanism (60) prevents the rotating sleeve (30) from moving when the needle protection sleeve (20) is in the first position, and an unlocked state in which the locking mechanism (60) allows the rotating sleeve (30) to carry out the helical movement so as to cause the needle protection sleeve (20) to move from the first position to the second position.

10. The patch-like medical device of claim 9, further comprising a trigger mechanism (80) operable by the patient, wherein the trigger mechanism (80) is configured to switch the locking mechanism (60) from the locked state to the unlocked state.

11. The patch-like medical device of claim 9 or 10, wherein the locking mechanism (60) comprises a latch (62) pivotably connected to the base (10), the latch (62) being engaged with the rotating sleeve (30) in the locked state to prevent the rotating sleeve (30) from moving and disengaged from the rotating sleeve (30) in the unlocked state to allow the rotating sleeve (30) to carry out the helical movement.

12. The patch-like medical device of claim 11, wherein the locking mechanism (60) comprises a lever element (63) being connected to the latch (62) for facilitating control of the latch (62), wherein the lever element (63) is movable between a first lever element position in which the lever element (63) prevents the latch (62) from disengaging from the rotating sleeve (30), and a second lever element position in which the lever element (63) allows the latch (62) to disengage from the rotating sleeve (30).

13. The patch-like medical device of claim 12, wherein the locking mechanism (60) further comprises a clamp element (64) configured for clamping the lever element (63) to prevent inadvertent motion of the lever element (63) and the latch (62), wherein the clamp element (64) is movable between a first clamp position in which the clamp element (64) fixes the lever element (63) with respect to the base (10) in the first lever element position, and a second clamp position in which the clamp element (64) releases the lever element (63) and allows the lever element (63) to move from the first lever element position to the second lever element position.

14. The patch-like medical device of claim 13, wherein the trigger mechanism comprises control circuitry (81) and an actuation mechanism (82) configured to mechanically actuate the clamp element (64) upon receiving an electrical signal from the control circuitry (81), causing the clamp element (64) to move from the first clamp position to the second clamp position so as to release the lever element (63).

## Patentansprüche

1. Pflasterartige medizinische Vorrichtung, umfassend:
eine Basis (10) mit einer Kontaktfläche (11), die so ausgebildet ist, dass sie auf die Haut eines Patienten gerichtet ist;
eine relativ zur Basis (10) fest positionierte Nadel (12), wobei die Nadel (12) eine Nadelspitze (121) aufweist, die aus der Kontaktfläche (11) herausragt und zum Durchstechen der Haut des Patienten ausgebildet ist;
eine entlang einer Mittelachse (A) von einer ersten Position in eine zweite Position bewegbare Nadelschutzhülse (20), wobei sich die Nadelschutzhülse (20) über die Nadelspitze (121) hinaus erstreckt, um in der ersten Position zu verhindern, dass die Nadelspitze (121) die Haut des Patienten durchsticht, und in der zweiten Position die Nadelspitze (121) freigibt, um das Durchstechen der Haut des Patienten zu ermöglichen;
**dadurch gekennzeichnet, dass** die pflasterartige medizinische Vorrichtung ferner umfasst:
eine entlang der Mittelachse (A) bewegbare Drehhülse (30), wobei die Drehhülse (30) in einem gewindeartigen Eingriff mit der Nadelschutzhülse (20) steht, so dass eine schraubenförmige Bewegung der Drehhülse (30) relativ zur Basis (10) bewirkt, dass sich die Nadelschutzhülse entlang der Mittelachse (A) von der ersten Position in die zweite Position bewegt;
eine Drehsicherungshülse (40), die entlang der Mittelachse (A) bewegbar ist, während sie in Bezug auf die Basis drehfest ist, wobei die Drehsicherungshülse (40) mit der Nadelschutzhülse (20) in Eingriff steht, so dass die Nadelschutzhülse (20) daran gehindert wird, sich in Bezug auf die Basis (10) zu drehen, wenn sich die Nadelschutzhülse (20) von der ersten Position in die zweite Position bewegt.

2. Pflasterartige medizinische Vorrichtung nach Anspruch 1,
wobei die Drehhülse (30) zumindest teilweise aus der Kontaktfläche (11) herausragt, wenn sich die Nadelschutzhülse (20) in der ersten Position befindet,
wobei sich die Drehhülse (30) während ihrer schraubenförmigen Bewegung entlang der Mittelachse (A) axial in Richtung der Kontaktfläche (11) zurückzieht, und wobei die Nadelschutzhülse (20) so ausgebildet ist, dass sie sich während der schraubenförmigen Bewegung der Drehhülse (30) relativ zu der Drehhülse (30) entlang der Mittelachse (A) axial bewegt.

3. Pflasterartige medizinische Vorrichtung nach Anspruch 1 oder 2,
wobei in der zweiten Position die Nadelschutzhülse (20), die Drehhülse (30) und die Drehsicherungshülse (40) jeweils zumindest teilweise, vorzugsweise vollständig, auf einer von der Kontaktfläche (11) abgewandten Seite in die pflasterartige medizinische Vorrichtung zurückgezogen sind.

4. Pflasterartige medizinische Vorrichtung nach einem der vorhergehenden Ansprüche,
wobei die Nadelschutzhülse (20) zumindest teilweise radial innerhalb der Drehhülse (30) aufgenommen ist,
wobei die Basis (10) auf einer von der Kontaktfläche abgewandten Seite eine Aussparung (13) definiert, wobei die Aussparung in radialer Richtung durch eine Seitenwand (133) begrenzt ist, und
wobei die Drehhülse (30) und die Seitenwand (133) in einem gewindeartigen Eingriff miteinander stehen.

5. Pflasterartige medizinische Vorrichtung nach einem der Ansprüche 1-3,
wobei die Drehhülse (30) zumindest teilweise radial innerhalb der Nadelschutzhülse (20) aufgenommen ist,
wobei die Basis (10) ein zentrales Führungselement (14) umfasst, das sich entlang der Mittelachse (A) erstreckt,
wobei das zentrale Führungselement (14) zumindest teilweise radial innerhalb der Drehhülse (30) aufgenommen ist,
und wobei die Drehhülse (30) und das zentrale Führungselement (14) in einem gewindeartigen Eingriff miteinander stehen.

6. Pflasterartige medizinische Vorrichtung nach einem der vorhergehenden Ansprüche,
wobei entweder die Nadelschutzhülse (20) oder die Drehsicherungshülse (40) eine Längsnut (23, 43) aufweist, die sich parallel zur Mittelachse (A) erstreckt, und wobei die jeweils andere der Nadelschutzhülse (20) und der Drehsicherungshülse (40) einen Führungsnocken (24, 42) aufweist, der so ausgebildet ist, dass er in der Längsnut (23, 43) gleitet, sodass die Schutzhülse (20) beim Bewegen von der ersten Position in die zweite Position daran gehindert wird, sich relativ zur Basis (10) zu drehen.

7. Pflasterartige medizinische Vorrichtung nach einem der vorhergehenden Ansprüche, ferner umfassend ein Befestigungselement, das fest an der Basis (10) angebracht ist und mit der Drehsicherungshülse (40) in Eingriff steht, so dass die Drehsicherungshülse daran gehindert wird, sich relativ zur Basis zu drehen, wobei das Befestigungselement vorzugsweise als Befestigungsring (70) ausgebildet ist.

8. Pflasterartige medizinische Vorrichtung nach einem der vorhergehenden Ansprüche, die ferner eine Feder (51) umfasst, die so ausgebildet ist, dass sie die schraubenförmige Bewegung der Drehhülse (30) antreibt, wobei die Feder (51) vorgespannt ist, wenn sich die Nadelschutzhülse (20) in der ersten Position befindet, wobei sich die Feder (51) zumindest teilweise entspannt, wenn sich die Nadelschutzhülse (20) von der ersten Position in die zweite Position bewegt.

9. Pflasterartige medizinische Vorrichtung nach einem der vorhergehenden Ansprüche, ferner umfassend einen Verriegelungsmechanismus (60), der einen verriegelten Zustand, in dem der Verriegelungsmechanismus (60) eine Bewegung der Drehhülse (30) verhindert, wenn sich die Nadelschutzhülse (20) sich in der ersten Position befindet, und einen entriegelten Zustand aufweist, in dem der Verriegelungsmechanismus (60) es der Drehhülse (30) ermöglicht, die schraubenförmige Bewegung auszuführen, um die Nadelschutzhülse (20) von der ersten Position in die zweite Position zu bewegen.

10. Pflasterartige medizinische Vorrichtung nach Anspruch 9, ferner umfassend einen vom Patienten betätigbaren Auslösemechanismus (80), wobei der Auslösemechanismus (80) so konfiguriert ist, dass er den Verriegelungsmechanismus (60) vom verriegelten Zustand in den entriegelten Zustand versetzt.

11. Pflasterartige medizinische Vorrichtung nach Anspruch 9 oder 10, wobei der Verriegelungsmechanismus (60) eine mit der Basis (10) schwenkbar verbundene Verriegelung (62) umfasst, wobei die Verriegelung (62) im verriegelten Zustand mit der Drehhülse (30) in Eingriff steht, um eine Bewegung der Drehhülse (30) zu verhindern, und im entriegelten Zustand von der Drehhülse (30) gelöst ist, um der Drehhülse (30) die Ausführung der schraubenförmigen Bewegung zu ermöglichen.

12. Pflasterartige medizinische Vorrichtung nach Anspruch 11, wobei der Verriegelungsmechanismus (60) ein Hebelelement (63) umfasst, das mit der Verriegelung (62) verbunden ist, um die Steuerung der Verriegelung (62) zu ermöglichen, wobei das Hebelelement (63) zwischen einer ersten Hebelelementposition, in der das Hebelelement (63) verhindert, dass sich die Verriegelung (62) von der Drehhülse (30) löst, und einer zweiten Hebelelementposition, in der das Hebelelement (63) ein Lösen der Verriegelung (62) von der Drehhülse (30) ermöglicht.

13. Pflasterartige medizinische Vorrichtung nach Anspruch 12, wobei der Verriegelungsmechanismus (60) ferner ein Klemmelement (64) umfasst, das zum Festklemmen des Hebelelements (63) ausgebildet ist, um eine unbeabsichtigte Bewegung des Hebelelements (63) und der Verriegelung (62) zu verhindern, wobei das Klemmelement (64) zwischen einer ersten Klemmposition, in der das Klemmelement (64) das Hebelelement (63) in Bezug auf die Basis (10) in der ersten Hebelelementposition fixiert, und einer zweiten Klemmposition, in der das Klemmelement (64) das Hebelelement (63) freigibt und es dem Hebelelement (63) ermöglicht, sich von der ersten Hebelelementposition in die zweite Hebelelementposition zu bewegen.

14. Pflasterartige medizinische Vorrichtung nach Anspruch 13, wobei der Auslösemechanismus eine Steuerschaltung (81) und einen Betätigungsmechanismus (82) umfasst, der so konfiguriert ist, dass er das Klemmelement (64) bei Empfang eines elektrischen Signals von der Steuerschaltung (81) mechanisch betätigt, wodurch das Klemmelement (64) von der ersten Klemmposition in die zweite Klemmposition bewegt wird, um das Hebelelement (63) freizugeben.

## Revendications

1. Un dispositif médical de type patch comprenant :
une base (10) comportant une surface de contact (11) configurée pour être orientée vers la peau d'un patient ;
une aiguille (12) positionnée de manière fixe par rapport à la base (10), l'aiguille (12) comportant une pointe d'aiguille (121) qui fait saillie à partir de la surface de contact (11) et qui est configurée pour percer la peau du patient ;
un manchon de protection d'aiguille (20) mobile le long d'un axe central (A) d'une première position à une deuxième position, le manchon de protection d'aiguille (20) s'étendant au-delà de la pointe d'aiguille (121) pour empêcher la pointe d'aiguille (121) de percer la peau du patient dans la première position, et exposant la pointe d'aiguille (121) pour permettre de percer la peau du patient dans la deuxième position ;
**caractérisé en ce que** le dispositif médical de type patch comprend en outre
un manchon rotatif (30) mobile le long de l'axe central (A), le manchon rotatif (30) étant en prise par filetage avec le manchon de protection d'aiguille (20) de telle sorte qu'un mouvement hélicoïdal du manchon rotatif (30) par rapport à la base (10) provoque le déplacement du manchon de protection d'aiguille le long de l'axe central (A) de la première position à la deuxième position ;
un manchon de verrouillage de rotation (40) mobile le long de l'axe central (A) tout en étant fixe en rotation par rapport à la base, le manchon de verrouillage de rotation (40) étant en prise avec le manchon de protection d'aiguille (20) de telle sorte que le manchon de protection d'aiguille (20) soit empêché de tourner par rapport à la base (10) lorsque le manchon de protection d'aiguille (20) se déplace de la première position à la deuxième position.

2. Le dispositif médical en forme de patch selon la revendication 1,
dans lequel le manchon rotatif (30) fait saillie au moins partiellement de la surface de contact (11) lorsque le manchon de protection d'aiguille (20) se trouve dans la première position,
dans lequel le manchon rotatif (30) se rétracte axialement vers la surface de contact (11) le long de l'axe central (A) pendant son mouvement hélicoïdal, et dans lequel le manchon de protection d'aiguille (20) est configuré pour se déplacer axialement par rapport au manchon rotatif (30) le long de l'axe central (A) pendant le mouvement hélicoïdal du manchon rotatif (30).

3. Le dispositif médical en forme de patch selon la revendication 1 ou 2,
dans lequel, dans la deuxième position, le manchon de protection d'aiguille (20), le manchon rotatif (30) et le manchon de verrouillage de rotation (40) sont chacun au moins partiellement, de préférence entièrement, rétractés à l'intérieur du dispositif médical en forme de patch sur un côté opposé à la surface de contact (11).

4. Le dispositif médical en forme de patch selon l'une quelconque des revendications précédentes,
dans lequel le manchon de protection d'aiguille (20) est au moins partiellement logé radialement à l'intérieur du manchon rotatif (30),
dans lequel la base (10) définit un évidement (13) sur un côté opposé à la surface de contact, l'évidement étant délimité par une paroi latérale (133) dans une direction radiale, et
dans lequel le manchon rotatif (30) et la paroi latérale (133) sont en prise par filetage l'un avec l'autre.

5. Le dispositif médical en forme de patch selon l'une quelconque des revendications 1 à 3,
dans lequel le manchon rotatif (30) est au moins partiellement logé radialement à l'intérieur du manchon de protection d'aiguille (20),
dans lequel la base (10) comprend un élément de guidage central (14) s'étendant le long de l'axe central (A),
dans lequel l'élément de guidage central (14) est au moins partiellement logé radialement à l'intérieur du manchon rotatif (30),
et dans lequel le manchon rotatif (30) et l'élément de guidage central (14) sont en prise par filetage l'un avec l'autre.

6. Le dispositif médical en forme de patch selon l'une quelconque des revendications précédentes,
dans lequel l'un parmi le manchon de protection d'aiguille (20) et le manchon de verrouillage de rotation (40) comprend une rainure longitudinale (23, 43) s'étendant parallèlement à l'axe central (A) et dans lequel l'autre parmi le manchon de protection d'aiguille (20) et le manchon de verrouillage de rotation (40) comprend une came de guidage (24, 42) configurée pour coulisser dans ladite rainure longitudinale (23, 43) de telle sorte que le manchon de protection (20) soit empêché de tourner par rapport à la base (10) lors du déplacement de la première position à la deuxième position.

7. Le dispositif médical en forme de patch selon l'une quelconque des revendications précédentes, comprenant en outre un élément de fixation qui est attaché de manière fixe à la base (10) et qui est en prise avec le manchon de verrouillage de rotation (40) de telle sorte que le manchon de verrouillage de rotation est empêché de tourner par rapport à la base, dans lequel l'élément de fixation est de préférence configuré comme une bague de fixation (70).

8. Le dispositif médical en forme de patch selon l'une quelconque des revendications précédentes, comprenant en outre un ressort (51) configuré pour entraîner le mouvement hélicoïdal du manchon rotatif (30), le ressort (51) étant précontraint lorsque le manchon de protection d'aiguille (20) se trouve dans la première position, dans lequel le ressort (51) se détend au moins partiellement lorsque le manchon de protection d'aiguille (20) se déplace de la première position vers la deuxième position.

9. Le dispositif médical en forme de patch selon l'une quelconque des revendications précédentes, comprenant en outre un mécanisme de verrouillage (60) présentant un état verrouillé dans lequel le mécanisme de verrouillage (60) empêche le manchon rotatif (30) de se déplacer lorsque le manchon de protection d'aiguille (20) se trouve dans la première position, et un état déverrouillé dans lequel le mécanisme de verrouillage (60) permet au manchon rotatif (30) d'effectuer le mouvement hélicoïdal de manière à amener le manchon de protection d'aiguille (20) à se déplacer de la première position vers la deuxième position.

10. Le dispositif médical en forme de patch selon la revendication 9, comprenant en outre
un mécanisme de déclenchement (80) actionnable par le patient, dans lequel le mécanisme de déclenchement (80) est configuré pour faire passer le mécanisme de verrouillage (60) de l'état verrouillé à l'état déverrouillé.

11. Le dispositif médical en forme de patch selon la revendication 9 ou 10, dans lequel le mécanisme de verrouillage (60) comprend un loquet (62) relié de manière pivotante à la base (10), le loquet (62) étant en prise avec le manchon rotatif (30) à l'état verrouillé pour empêcher le manchon rotatif (30) de se déplacer et désengager du manchon rotatif (30) à l'état déverrouillé pour permettre au manchon rotatif (30) d'effectuer le mouvement hélicoïdal.

12. Le dispositif médical en forme de patch selon la revendication 11, dans lequel le mécanisme de verrouillage (60) comprend un élément de levier (63) relié au loquet (62) pour faciliter la commande du loquet (62), l'élément de levier (63) étant mobile entre une première position de l'élément de levier dans laquelle l'élément de levier (63) empêche le loquet (62) de se désengager du manchon rotatif (30), et une deuxième position de l'élément de levier dans laquelle l'élément de levier (63) permet au loquet (62) de se désengager du manchon rotatif (30).

13. Le dispositif médical en forme de patch selon la revendication 12, dans lequel le mécanisme de verrouillage (60) comprend en outre un élément de serrage (64) configuré pour serrer l'élément de levier (63) afin d'empêcher tout mouvement involontaire de l'élément de levier (63) et du loquet (62), dans lequel l'élément de serrage (64) est mobile entre une première position de serrage dans laquelle l'élément de serrage (64) fixe l'élément de levier (63) par rapport à la base (10) dans la première position de l'élément de levier, et une deuxième position de serrage dans laquelle l'élément de serrage (64) libère l'élément de levier (63) et permet à l'élément de levier (63) de se déplacer de la première position de l'élément de levier vers la deuxième position de l'élément de levier.

14. Le dispositif médical en forme de patch selon la revendication 13, dans lequel le mécanisme de déclenchement comprend un circuit de commande (81) et un mécanisme d'actionnement (82) configuré pour actionner mécaniquement l'élément de serrage (64) lors de la réception d'un signal électrique provenant du circuit de commande (81), ce qui provoque le déplacement de l'élément de serrage (64) de la première position de serrage à la deuxième position de serrage de manière à libérer l'élément de levier (63).
